# EUROPEAN PATENT APPLICATION

(11) **EP 3 666 940 A1**
(43) Date of publication of application: **17.06.2020**
(21) Application number: 18212764.7
(22) Date of filing: 14.12.2018
(51) Int. Cl.: D01F 6/18, D01D 5/00

(54) **METHOD OF PRODUCING POLY(ALKYL CYANOACRYLATE) BASED NANO/MICROFIBERS AND USES THEREOF**

(71) Applicant: Charité - Universitätsmedizin Berlin, 10117 Berlin (DE); Universidad de La Habana, La Habana 10400 (CU); Wiwex GmbH, 10178 Berlin (DE)
(72) Inventor: MÜLLER, Wolf-Dieter, 12524 Berlin (DE); ALVAREZ BRITO, Ruben, La Habana (CU); RAMOS CARRILES, Yaquelin, 10900 La Habana (CU); HANSEN, Jan, 12167 Berlin (DE)
(74) Representative: Grünecker Patent- und Rechtsanwälte PartG mbB

(57) **Abstract**

The present invention relates to the field of biomaterials, more particularly to the field of poly(alkyl cyanoacrylate) based nano- and microfibers. The present invention provides a novel method for producing ready-to-use poly(alkyl cyanoacrylate) based nano/microfibers, wherein the method comprises electrospinning of poly(alkyl cyanoacrylate) homopolymers or copolymers generated by anionic polymerization of alkyl cyanoacrylate monomers or oligomers and characterized by a specific polydispersity index. Accordingly, the present invention also provides novel ready-to-use nano/microfibers obtainable by the method as well as uses thereof, including (therapeutic) biomedical applications such as wound healing, drug delivery and tissue regeneration and engineering.

## Description

### TECHNICAL FIELD

The present invention relates to the field of biomaterials, more particularly to the field of poly(alkyl cyanoacrylate) based nano- and microfibers. The present invention provides a novel method of producing ready-to-use poly(alkyl cyanoacrylate) based nano/microfibers, wherein the method comprises electrospinning of poly(alkyl cyanoacrylate) homopolymers or copolymers generated by anionic polymerization of alkyl cyanoacrylate monomers or oligomers and characterized by a specific polydispersity index. Accordingly, the present invention also provides novel ready-to-use nano/microfibers obtainable by the method as well as uses thereof, including (therapeutic) biomedical applications such as wound healing, drug delivery and tissue regeneration and tissue engineering.

### BACKGROUND

Poly(alkyl cyanoacrylate) polymers (PACA polymers) can be generated by direct Knoevenagel condensation of the respective alkyl cyanoacetates (ACAs) and formaldehyde (Nicolas and Couvreur, 2009, Synthesis of Poly(alkyl cyanoacrylate)-based Colloidal Nanomedicines. Wiley Interdiscip. Rev. Nanomed. Nanobiotechnol., Vol. 1(1): 111-127). PACA polymers have been studied for use in controlled drug delivery systems, which are produced by polymerization of alkyl cyanoacrylates or by precipitation of preformed PACAs from their organic solvents. Different techniques are known for producing PACA-based nanofibers, one of them being electrospinning (Teo et al., 2011, Technological Advances in Electrospinning of Nanofibers, Sci. Technol. Adv. Mater., Vol. 12(1):013002, 19 pp.). Briefly, the electrospinning technique uses electrical force to draw charged threads of polymer solutions on a collector with fiber diameters up to the nanometer range. One of the simplest electrospinning machines consists of a high voltage power supply, an injection pump system, a syringe with a needle, and a collector. WO 2014/184761 relates to a process for the production of poly cyanoacrylate fibers using electrospinning, and describes the formation of cyanoacrylate polymers in a dipolar aprotic solvent, which performs the dual function of being solvent for the cyanoacrylate monomer and catalyst for initiating polymerization.

PACA-based electro-spun nanofibers are considered biocompatible and form the scaffold of, e.g., nanofiber meshes that can be used in various biomedical applications. US 2014/0205971 A1 describes the electrospinning of nanofibers made from other synthetic polymers such as polycaprolactone (PCL) and poly(lactic-co-glycolic) acid (PLGA), and their use for bone grafting, tissue growth and regeneration in dental implants. Dental implants have become part of routine dentistry, and attention has been paid to soft tissue healing around implants accordingly. However, to date dental implant surgery is still associated with complications like material-related wound dehiscence.

There is a constant need for novel methods for producing PACA-based nano/microfibers that can serve as scaffolds for novel ready-to-use products like nanofiber meshes and their use in biomedical applications. Due to limitations in the art, in particular with regard to wound dressing-related complications in dental implant surgery, the present invention addresses needs in the art by providing a novel method of producing ready-to-use poly(alkyl cyanoacrylate) based nano/microfibers, as well as nano/microfibers obtainable by the method and uses thereof in biomedical applications.

### SUMMARY OF THE INVENTION

The present disclosure provides the following [1] to [15], without being specifically limited thereto:
[1] A method of producing ready-to-use poly(alkyl cyanoacrylate) based nano/microfibers, the method comprising:
   (a) providing a poly(alkyl cyanoacrylate) homopolymer or poly(alkyl cyanoacrylate) copolymer obtained by anionic polymerization of one or more alkyl cyanoacrylate (ACA) monomers or one or more ACA oligomers, wherein the alkyl substituent of the ACA monomer or the ACA oligomer may optionally be substituted, and wherein the ratio of the weight-average molecular weight (M_{w}) to the number-average molecular weight (Mₙ) of the poly(alkyl cyanoacrylate) homopolymer or poly(alkyl cyanoacrylate) copolymer is from 1 to 1.7;
   (b) dissolving the poly(alkyl cyanoacrylate) homopolymer or poly(alkyl cyanoacrylate) copolymer of step (a) in a solvent;
   (c) electrospinning the solution obtained in step b) to obtain poly(alkyl cyanoacrylate)-based nano/microfibers on a collector; and
   (d) removing the poly(alkyl cyanoacrylate) based nano/microfibers from the collector to obtain ready-to-use poly(alkyl cyanoacrylate) based nano/microfibers.
[2] The method of [1], wherein the poly(alkyl cyanoacrylate) copolymer in step (a) comprises (i) at least two different ACA monomer species, (ii) at least two alkyl cyanoacrylate oligomers comprising at least two different ACA monomer species, or (iii) at least one poly(alkyl cyanoacrylate) (PACA) polymer and at least one non-PACA polymer.
[3] The method of [1] or [2], wherein the one or more ACA monomers, or the at least two different ACA monomer species, are selected from any of n-butyl-2-cyanoacrylate (BCA), isobutyl-2-cyanoacrylate (IBCA), n-pentyl-2-cyanoacrylate (PCA), iso-pentyl-2-cyanoacrylate (IPCA), n-hexyl-2-cyanoacrylate (HCA), iso-hexyl-2-cyanoacrylate (IHCA), cyclo-hexyl-2-cyanoacrylate (CHCA), n-heptyl-2-cyanoacrylate (HepCA), iso-heptyl-2-cyanoacrylate (IHepCA), n-octyl-2-cyanoacrylate (OCA), iso-octyl-2-cyanoacrylate (IOCA), butyl-lactoyl-2-cyanoacrylate (BLCA), and mixtures thereof.
[4] The method of [1] or [2], wherein the one or more ACA oligomers are selected from oligomers of any of n-butyl-2-cyanoacrylate (BCA), oligomers of iso-butyl-2-cyanoacrylate (IBCA), oligomers of n-pentyl-2-cyanoacrylate (PCA), oligomers of iso-pentyl-2-cyanoacrylate (IPCA), oligomers of n-hexyl-2-cyanoacrylate (HCA), oligomers of iso-hexyl-2-cyanoacrylate (IHCA), oligomers of cyclo-hexyl-2-cyanoacrylate (CHCA), oligomers of n-heptyl-2-cyanoacrylate (HepCA), oligomers of iso-heptyl-2-cyanoacrylate (IHepCA), oligomers of n-octyl-2-cyanoacrylate (OCA), oligomers of iso-octyl-2-cyanoacrylate (IOCA), oligomers of butyl-lactoyl-2-cyanoacrylate (BLCA), and mixtures thereof.
[5] The method of any of [1] to [4], wherein the solvent in step (b) is selected from any of acetone, ethanol, acetic acid, formic acid, ethyl acetate, dimethyl sulfoxide, dimethyl formamide, butyl acetate, isobutyl acetate, and mixtures thereof.
[6] The method of any of [1] to [5], wherein at least one non-PACA polymer is added to the solution obtained in step (b) prior to electrospinning. In various embodiments, the at least one non-PACA polymer is at least one non-PACA homopolymer and/or at least one non-PACA copolymer.
[7] The method of any one of [2] to [6], wherein the at least one non-PACA polymer is selected from any of poly(lactic-co- glycolic acid) (PLGA), poly(ε-caprolactone) (PCL), poly(ethyelenglycol) (PEG), poly(lactic acid) (PLA), chitosan (CH), hyaluronic acid (HA), and dextran (Dex).
[8] The method of any of [1] to [7], wherein the ready-to-use nano/microfibers obtained in step (d) are not comprised by a support.
[9] The method of any of [1] to [8], wherein the ratio of the weight average molecular weight to the number average molecular weight is from 1.1 to 1.5; or wherein the ratio of the weight average molecular weight to the number average molecular weight is from 1.2 to 1.4.
[10] Ready-to-use nano/microfibers obtainable by or obtained by the method of any of [1] to [9].
[11] The ready-to-use nano/microfibers of [10], wherein the fiber average diameter is from 100 nm to 5 micrometer.
[12] The ready-to-use nano/microfibers of [10], wherein the nano/microfibers is immobilized with, or has encapsulated, at least one of a therapeutic agent, antibiotic, antiviral agent, chemotherapeutic agent, analgesic and analgesic combinations, anti-inflammatory agent, vitamin, sedative, radiopharmaceutical, metal particles, metal alloy particles, metal oxide particles, hydroxyapatite, sodium alginate, stains, cells, protein, peptides, nucleic acids, nucleic acid analogs, nucleotides or oligonucleotides, peptide nucleic acids, aptamers, antibodies or fragments or portions thereof, antigens or epitopes, hormones, hormone antagonists, growth factors or recombinant growth factors and fragments and variants thereof, cell attachment mediators, cytokines, enzymes, or a mixture thereof.
[13] The ready-to-use nano/microfibers of [10], wherein the nano/microfibers further comprise a cell growth medium.
[14] Use of the ready-to-use nano/microfibers of [10] in dental applications, for wound dressing/healing, tissue regeneration/engineering, organ protection, implant's coating, or controlled drug delivery.
[15] A nanofiber mesh comprising the ready-to-use nano/microfibers of any of [10] to [13], wherein the surface of the nanofiber mesh is modified chemically and/or physically for biomedical applications, preferably wherein the surface is modified by encapsulation and/or immobilization of a therapeutic agent and/or biological material.
Further aspects and embodiments of the invention are set forth in the following detailed description.

### BRIEF DESCRIPTION OF THE DRAWINGS

**Figure 1** illustrates an exemplary electrospinning set up. A: high voltage power supply, B: injection system pump, C: collector connected to the earth.
**Figure 2** shows an exemplary injection system pump, wherein the syringe is coupled to the needle, and the collector is connected to the earth.
**Figure 3** shows electro-spun meshes based on PACAs collected over copper meshes used as collectors.
**Figure 4** shows electro-spun meshes based on PACAs collected over different collectors. A: copper rings, B: aluminum foil, C: paper.
**Figure 5** shows fluorescence pictures with different magnifications of human gingival fibroblasts (HGFib) growing on PBCA meshes during 23 days of incubation at 37°C and 5% (V/V) of CO₂. A: after 3 days of incubation, B: after 7 days of incubation, C: after 14 days of incubation, D: after 23 days of incubation. Magnifications for each of A-D: left bar 500 nm, middle bar 200 nm, right bar 100 nm.
**Figure 6** shows SEM (scanning electron microscope) pictures of meshes based on PACAs. A and B: PACAs with different molecular weighs, bar = 5 µm in both pictures of A, and for B: bar in the two pictures at the top 5 µm, bar in the two pictures at the bottom 2 µm, bar in the left picture in the middle 10 µm, bar in the right picture in the middle 5 µm; C: PACA/PLGA; D: PACA/5FU; E: PACA/PACA-co-CS.
**Figure 7** shows the GPC (gel permeation chromatography) results of A: powder of PACA; B: powder of PACA-co-CS; C: electro-spun mesh of PACA/PACA-co-CS.

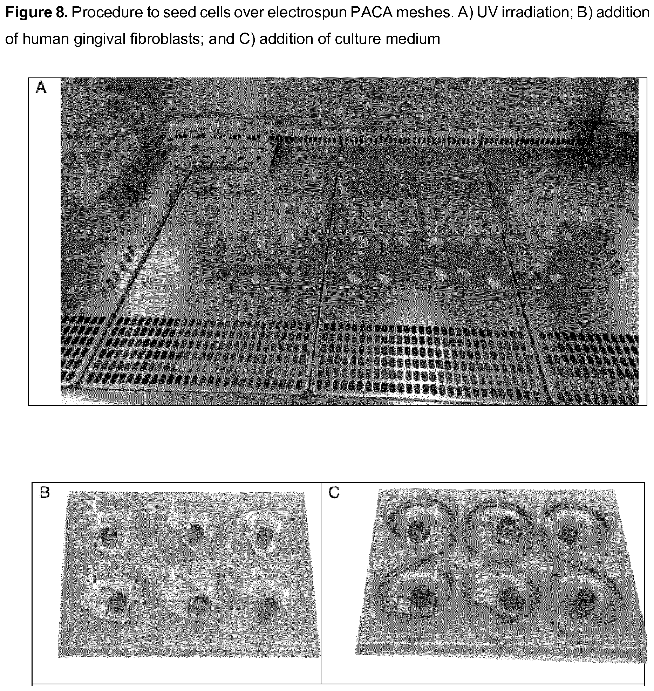

**Figure 8** illustrates the procedure of seeding cells over electro-spun PACAs meshes. A: UV irradiation; B: addition of human gingival fibroblasts; C: addition of cell culture medium.
**Figure 9** shows the release of 5FU from a PACA/5FU mesh incubated in PBS, pH 7.4 at 37°C. Three different samples tested, m1, m2, m3, having different initial weight of 5FU encapsulated in the respective mesh: m1 = 7.30 mg, m2 = 6.67 mg, m3 = 6.12 mg. Samples were taken from the same PACA/5FU mesh produced. Fig. 9 shows a consistent drug release pattern with up to 51-55% within 30 days in PBS medium. At day 96, the drug release was 79-87% of the total drug content for all samples.
**Figure 10** is a SEM picture of a PBCA mesh before (A) and after (B) 23 days of incubation with HGFib. Mesh cleaned with water.
**Figure 11** is a SEM picture of PACA-based meshes after being produced at year 0 (A1 and B1), and after one (1) year of storage under normal conditions (A2 and B2). A1 and A2: flat fibers; B1 and B2: round fibers. Normal conditions: ambient temperature and pressure (25°C, 1 atm).
**Figure 12** is a SEM picture of PLGA- and PACA/PLGA-based meshes after being produced at year 0 (A1 and B1), and after one (1) year of storage under normal conditions (A2 and B2). Normal conditions: ambient temperature and pressure (25°C, 1 atm).
**Figure 13** illustrates confocal laser scanning microscopy of a PACA mesh stained with D&C Violet #2.

### DETAILED DESCRIPTION OF THE INVENTION

The present invention demonstrates that meshes or mesh networks of PACA nano/microfibers can be produced by electrospinning of PACA solutions. Different types of meshes have been produced, as shown in the Examples and the accompanying drawings. The present invention provides as a general concept a method of producing poly(alkyl cyanoacrylate) (PACA)-based **nano/microfibers and/or PACA-based nano/microfiber meshes**, the method comprising:
(a) **providing** a poly(alkyl cyanoacrylate) homopolymer and/or poly(alkyl cyanoacrylate) copolymer, wherein the ratio of the weight-average molecular weight (M_{w}) to the number-average molecular weight (Mₙ) of the poly(alkyl cyanoacrylate) homopolymer and/or poly(alkyl cyanoacrylate) copolymer is from 1 to 1.7;
(b) dissolving the poly(alkyl cyanoacrylate) homopolymer or poly(alkyl cyanoacrylate) copolymer of step (a) in a solvent; and
(c) electrospinning the solution obtained in step (b) to obtain poly(alkyl cyanoacrylate)-based nano/microfibers, preferably electrospinning the solution obtained in step (b) to obtain poly(alkyl cyanoacrylate)-based nano/microfibers on a collector.

In various embodiments, the alkyl substituent of the poly(alkyl cyanoacrylate) homopolymer and/or poly(alkyl cyanoacrylate) copolymer may optionally be substituted, in accordance with substitutions which are described elsewhere herein. Furthermore, in various embodiments, the poly(alkyl cyanoacrylate) homopolymer and/or poly(alkyl cyanoacrylate) copolymer in step (a) is **obtainable by, or is obtained by**, ionic polymerization of one or more alkyl cyanoacrylate (ACA) monomers and/or one or more ACA oligomers, wherein the alkyl substituent of the ACA monomers and/or the ACA oligomers may optionally be substituted. In various embodiments, the ionic polymerization is anionic polymerization, or is cationic polymerization. Preferably, the ionic polymerization is an anionic polymerization. The method may comprise a further step (d) of removing the poly(alkyl cyanoacrylate) based nano/microfibers from the collector to obtain poly(alkyl cyanoacrylate) based nano/microfibers and/or PACA-based nano/microfiber meshes. Accoringly, the

In various aspects, the method of producing poly(alkyl cyanoacrylate)-based nano/microfibers is a method of producing poly(alkyl cyanoacrylate)-based **nano/microfibers and/or PACA-based nano/microfiber meshes**, the method comprising:
(a) providing a poly(alkyl cyanoacrylate) homopolymer and/or poly(alkyl cyanoacrylate) copolymer obtained by ionic polymerization of one or more alkyl cyanoacrylate (ACA) monomers and/or one or more ACA oligomers, wherein the alkyl substituent of the ACA monomers and/or the ACA oligomers may optionally be substituted, and wherein the ratio of the weight-average molecular weight (M_{w}) to the number-average molecular weight (Mₙ) of the poly(alkyl cyanoacrylate) homopolymer or poly(alkyl cyanoacrylate) copolymer is from 1 to 1.7;
(b) dissolving the poly(alkyl cyanoacrylate) homopolymer and/or poly(alkyl cyanoacrylate) copolymer of step (a) in a solvent;
(c) electrospinning the solution obtained in step (b) to obtain a poly(alkyl cyanoacrylate)-based nano/microfiber mesh on a collector; and
(d) removing the poly(alkyl cyanoacrylate) based nano/microfiber meshes from the collector to obtain a poly(alkyl cyanoacrylate) based nano/microfibers and/or PACA-based nan/microfiber meshes. In various embodiments, the ionic polymerization in step (a) is anionic polymerization, or is cationic polymerization. Preferably, ionic polymerization is anionic polymerization.

In various embodiments, a PACA-based nano/microfiber mesh of the present invention may be described as PACA-based nano/microfiber network or PACA-based nano/microfiber mesh network. The said terms may be used interchangeably herein. The term "mesh" (or "network" or "network mesh"), also known as a scaffold or mat, may be considered to relate to a three-dimensional frame, porous or even highly porous, with well-interconnected pores that can be invaded and populated by cells, or that can have various functions or properties such as acting as protective barrier, offering or providing an excellent environment for healing, offering a high surface-to-volume ratio, allowing exuding fluid from wounds, allowing gas permeation, inhibiting the exogenous microorganism invasion to wounds, assisting the control of fluid drainage, and serving as delivery platform for any kind of agents including therapeutic agents, other chemical substances, compounds, or biological material.

As described herein, the term "PACA" is used as an abbreviation for the term "poly(alkyl cyanoacrylate)". Likewise, as described herein, the term "ACA" is used as an abbreviation for the term "alkyl cyanoacrylate".

Furthermore, the term "PACA-based nano/microfibers" encompasses "PACA-based nanofibers and/or PACA-based microfibers". Likewise, the term "PACA-based nano/microfiber mesh(es)" encompasses "PACA-based nanofiber mesh(es)" and/or "PACA-based microfiber mesh(es)".

As used herein, the term "PACA-based nanofiber(s)" means that the PACA-based fibers have an average diameter in the nanometer size range. In various aspects, "PACA-based nanofiber(s)" means that the nanofiber(s) have an (average) diameter in the range from about 1 nm to about 999 nm, which includes (average) diameters in the range between 0,01 nm to 1 nm. In various embodiments, the "PACA-based nanofiber(s)" have an (average) diameter in the range from about 100 nm to about 900 nm. In various other embodiments, the "PACA-based nanofiber(s)" have an (average) diameter in the range from about 200 nm to about 800 nm. In further embodiments, the "PACA-based nanofiber(s)" have an (average) diameter in the range from about 300 nm to about 700 nm. In still other embodiments, the "PACA-based nanofiber(s)" have an (average) diameter in the range from about 400 nm to about 600 nm. In various embodiments, the "PACA-based nanofiber(s)" have an (average) diameter of about 650 ± 200 nm or about 700 ± 230 nm. In various other embodiments, the "PACA-based nanofiber(s)" have an (average) diameter of about 675 ± 230 nm. In still other embodiments, the "PACA-based nanofiber(s)" have an (average) diameter of about 700 ± 200 nm or about 700 ± 230 nm.

Likewise, as used herein, the term "PACA-based microfiber(s)" means that the PACA-based fibers have an average diameter in the micrometer size range. In various aspects, "PACA-based microfiber(s)" means that the microfiber(s) have an (average) diameter in the range from about 1 µm to about 1,000 µm, which includes (average) diameters in the range between 0,01 µm to 1 µm. In various embodiments, the "PACA-based microfiber(s)" have an (average) diameter in the range from about 100 µm to about 900 µm. In various other embodiments, the "PACA-based microfiber(s)" have an (average) diameter in the range from about 200 µm to about 800 µm. In further embodiments, the "PACA-based microfiber(s)" have an (average) diameter in the range from about 300 µm to about 700 µm. In still other embodiments, the "PACA-based microfiber(s)" have an (average) diameter in the range from about 400 µm to about 600 µm. In various embodiments, the "PACA-based microfiber(s)" have an (average) diameter of about 500 ± 200 µm or about 500 ± 250 nm. In various other embodiments, the "PACA-based microfiber(s)" have an (average) diameter of about 10 ± 5 µm. In still other embodiments, the "PACA-based microfiber(s)" have an (average) diameter of about 2 ± 1 µm. In various embodiments, the "PACA-based nano/microfiber(s)" have an (average) diameter in the range from about 600 nm to about 1.400 nm, preferably in the range from about 700 nm to about 1.300 nm, more preferably in the range from about 800 nm to about 1.200 nm, still more preferably in the range from about 900 nm to about 1.100 nm. In various embodiments, the "PACA-based nano/microfiber(s)" have an (average) diameter in the range from about 1,000 ± 400 nm, or in the range from about 1,000 ± 250 nm. In various other embodiments, the "PACA-based nano/microfiber(s)" have an (average) diameter in the range from about 1,000 ± 300 nm, preferably in the range from about 1,000 ± 250 nm, more preferably in the range from about 1,000 ± 200 nm, or even in the range from about 1,000 ± 100 nm.

In various aspects, the methods for producing PACA-based **nano/microfibers or PACA-based nano/microfiber meshes**, respectively, may comprise a step (a) as follows:
(a) **generating (or obtaining)** a poly(alkyl cyanoacrylate) homopolymer and/or poly(alkyl cyanoacrylate) copolymer **by** ionic polymerization of one or more alkyl cyanoacrylate (ACA) monomers and/or one or more ACA oligomers, wherein the alkyl substituent of the ACA monomers and/or the ACA oligomers may optionally be substituted, and wherein the ratio of the weight-average molecular weight (M_{w}) to the number-average molecular weight (Mₙ) of the poly(alkyl cyanoacrylate) homopolymer and/or poly(alkyl cyanoacrylate) copolymer is from 1 to 1.7. Accordingly, step (b) then reads as follows: (b) dissolving the poly(alkyl cyanoacrylate) homopolymer and/or poly(alkyl cyanoacrylate) copolymer generated (or obtained) in step (a) in a solvent. In various embodiments, the ionic polymerization is anionic polymerization, or is cationic polymerization: Preferably, ionic polymerization is anionic polymerization.

In step (b) of the methods in accordance with the present invention, any suitable solvent can be employed as long as the poly(alkyl cyanoacrylate) homopolymer or poly(alkyl cyanoacrylate) copolymer is sufficiently solved therein. The solutions are obtained by dissolving the PACA homopolymers or PACA copolymers therein, preferably by mixing using magnetic stirring until the homopolymer or copolymer is completely dissolved and a clear solution is obtained. In case more than one polymer is used for electrospinning (e.g., in case a non-PACA polymer is added to the solution obtained in step (b) of the methods described herein prior to electrospinning), the respective polymeric solutions may be prepared independently, followed by mixing them. Alternatively, a solution comprising only one PACA homopolymer or PACA copolymer may be prepared, followed by the addition of further components.

In various embodiments of the present invention, the poly(alkyl cyanoacrylate) homopolymer and/or poly(alkyl cyanoacrylate) copolymer may be obtainable by, or may be obtained, or may be generated by, (direct) Knoevenagel condensation between the corresponding alkyl cyanoacrylate(s) and formaldehyde, and/or by ionic polymerization of one or more ACA monomers and/or one or more ACA oligomers, and/or by ionic polymerization of one or more ACA monomers with one or more non-PACA polymers. Such non-PACA polymers may catalyze or initiate the ionic polymerization of the ACA monomers by virtue of ionic groups of the non-PACA polymers, *i.e*., the ionic groups serve as the initiator of the anionic polymerization of the ACA monomers. In various embodiments, the aforementioned ionic polymerization is anionic polymerization, or is cationic polymerization. Preferably, ionic polymerization is anionic polymerization. In accordance with the aforementioned, the methods described herein may encompass in step (a), (i) (direct) Knoevenagel condensation between the corresponding alkyl cyanoacrylate(s) and formaldehyde, and/or (ii) ionic (anionic or cationic) polymerization of one or more ACA monomers and/or one or more ACA oligomers, and/or (iii) by ionic (anionic or cationic) polymerization of one or more ACA monomers with one or more non-PACA polymers.

Further disclosed herein is a method of producing (ready-to-use) poly(alkyl cyanoacrylate) based nano/microfibers and/or PACA-based nano/microfiber meshes, the method comprising:
(a) providing a poly(alkyl cyanoacrylate) homopolymer and/or poly(alkyl cyanoacrylate) copolymer, wherein the ratio of the weight-average molecular weight (M_{w}) to the number-average molecular weight (Mₙ) of the poly(alkyl cyanoacrylate) homopolymer and/or poly(alkyl cyanoacrylate) copolymer is from 1 to 1.7;
(b) dissolving the poly(alkyl cyanoacrylate) homopolymer and/or poly(alkyl cyanoacrylate) copolymer of step (a) in a solvent; and
(c) electrospinning the solution obtained in step (b) to obtain poly(alkyl cyanoacrylate)-based nano/microfibers and/or PACA-based nano/microfiber meshes, preferably electrospinning the solution obtained in step (b) to obtain poly(alkyl cyanoacrylate)-based nano/microfibers and/or PACA-based nano/microfiber meshes on a collector.

The aforementioned method may comprise a further a step (d) of removing the poly(alkyl cyanoacrylate) based nano/microfibers and/or PACA-based nano/microfiber meshes from the collector to obtain ready-to-use poly(alkyl cyanoacrylate) based nano/microfibers and/or PACA-based nano/microfiber meshes. In various embodiments, the poly(alkyl cyanoacrylate) copolymer comprises, or is formed by, (i) one or more PACA polymers, and (ii) one or more non-PACA polymers. The non-PACA polymer may be any non-PACA polymer as described elsewhere herein. Likewise, the PACA polymer may be any PACA polymer as described elsewhere herein. In various embodiments, the poly(alkyl cyanoacrylate) copolymer, which comprises, or which is formed by, (i) one or more PACA polymers, and (ii) one or more non-PACA polymers, is obtainable by, or is obtained by, or is generated by, ionic polymerization of (i) one or more alkyl cyanoacrylate (ACA) monomers (or ACA oligomers) and (ii) one or more non-PACA polymers. The ionic polymerization may be anionic polymerization, or may be cationic polymerization. Preferably, the ionic polymerization is anionic polymerization. The alkyl substituent of the ACA monomers and/or the ACA oligomers may optionally be substituted. In various embodiments, the ionic (anionic or cationic) polymerization may be initiated by an initiator, which may be an initiator as described elsewhere herein. In various preferred embodiments, the ionic (anionic or cationic) polymerization is initiated by the one or more non-PACA polymers. The present inventors have surprisingly found that ACA monomers can be polymerized using the ionic group of a non-PACA polymer as initiator for the polymerization. This allows for the production of copolymers, which may also be called graft copolymers. The present disclosure specifically encompasses (ready-to-use) PACA-based nano/microfibers (and/or PACA-based nano/microfiber meshes) obtainable by, or obtained by, a method of producing such grafted copolymers as described herein.

Further disclosed herein is a method of producing (ready-to-use) poly(alkyl cyanoacrylate) based nano/microfibers and/or PACA-based nano/microfiber meshes, the method comprising the steps:
(a) providing a poly(alkyl cyanoacrylate) copolymer comprising, or formed by, a PACA polymer and a non-PACA polymer,
(b) dissolving the poly(alkyl cyanoacrylate) copolymer of step (a) in a solvent; and
(c) electrospinning the solution obtained in step (b) to obtain poly(alkyl cyanoacrylate)-based nano/microfibers and/or PACA-based nano/microfiber meshes, preferably electrospinning the solution obtained in step (b) to obtain poly(alkyl cyanoacrylate)-based nano/microfibers and/or PACA-based nano/microfiber meshes on a collector. In preferred embodiments, the ratio of the weight-average molecular weight (M_{w}) to the number-average molecular weight (Mₙ) of the poly(alkyl cyanoacrylate) copolymer is from 1 to 1.7. The method may comprise a further step
(d) of removing the poly(alkyl cyanoacrylate) based nano/microfibers and/or PACA-based nano/microfiber meshes from the collector to obtain ready-to-use poly(alkyl cyanoacrylate) based nano/microfibers and/or PACA-based nano/microfiber meshes. In various embodiments, the poly(alkyl cyanoacrylate) copolymer comprises, or is formed by, (i) one or more PACA polymers, and (ii) one or more non-PACA polymers. In preferred embodiments, the PACA copolymer comprises (i) one or more PACA homopolymers and (ii) one or more non-PACA polymers. The non-PACA polymer may be any non-PACA polymer as described elsewhere herein, including any non-PACA homopolymers and any non-PACA copolymers described elsewhere herein. Likewise, the PACA (homo)polymer may be any PACA (homo)polymer as described elsewhere herein. In various embodiments, the PACA copolymer, which comprises, or which is formed by, one or more PACA (homo)polymers, and one or more non-PACA polymers, is obtainable by, or is obtained by, or is generated by, ionic polymerization of one or more alkyl cyanoacrylate (ACA) monomers (or ACA oligomers) and one or more non-PACA polymers. The ionic polymerization may be anionic polymerization, or may be cationic polymerization. Preferably, the ionic polymerization is anionic polymerization. The alkyl substituent of the ACA monomers and/or the ACA oligomers may optionally be substituted. In various embodiments, the ionic polymerization may be initiated by an initiator, which may be an initiator as described elsewhere herein. In various preferred embodiments, the ionic (anionic and cationic) polymerization is initiated by the one or more non-PACA polymers. The present inventors have surprisingly found that ACA monomers can be polymerized using the ionic group of a non-PACA polymer as initiator for the polymerization. This allows for the production of copolymers, which may also be called graft copolymers. The present disclosure specifically encompasses (ready-to-use) PACA-based nano/microfibers (and/or PACA-based nano/microfiber meshes) obtainable by, or obtained by, a method of producing such grafted copolymers as described herein.

In various embodiments, the PACA-based **nano/microfibers** or PACA-based **nano/microfiber meshes** produced are ready-to-use PACA-based nano/microfibers or ready-to-use PACA-based nano/microfiber meshes, respectively. In various embodiments, the PACA-based **nano/microfibers** or PACA-based **nano/microfiber meshes** produced are ready-to-use PACA-based nano/microfibers or ready-to-use PACA-based nano/microfiber meshes, respectively, because the one or more PACA homopolymers and/or one or more PACA copolymers are dissolved in a solvent (see step (b) of the methods described above), which is biocompatible. In various preferred embodiments, the biocompatible solvent is biocompatible with skin, bone and/or tissue. The terms "skin", "bone" or "tissue" as used herein encompass animal and human skin, bone or tissue. In various embodiments, the term "biocompatible solvent" means a solvent, which is known to be employed in the manufacture of drug substances, excipients, and drug products because the solvent is a non-toxic solvent, and/or because the solvent does not have any deleterious environmental effect. Accordingly, in various embodiments, the "biocompatible solvent" is a non-toxic solvent and/or does not have any deleterious environmental effect. Preferably, the solvent used for dissolving the one or more PACA homopolymers and/or one or more PACA copolymers can be regarded as less toxic and of lower risk to human health as compared to solvents, which should not be employed in the manufacture of drug substances, excipients, and drug products ("Class 1 solvents"), and as compared to solvents, which should be limited in pharmaceutical products because of their inherent toxicity ("Class 2 solvents"). Accordingly, in the present invention, the solvent used for dissolving the one or more PACA (homo)polymers and/or one or more PACA copolymers is a "Class 3 solvent", *i.e*., a solvent, which can be regarded as less toxic and of lower risk to human health as compared to solvents of Class 1 or 2. Solvents of Class 1, 2 and 3 are known to the skilled person from guidance for industry provided by, e.g., the U.S. FDA (Food and Drug Administration). See in particular the Human Use guidance for industry "*Q3C Impurities: Residual Solvents"* (Revision 3 of June 2017), which makes recommendations as to what amounts residual solvents are considered safe in pharmaceuticals. The solvents of "Class 3" encompass acetic acid, acetone, anisole, 1-butanol, 2-butanol, butyl acetate, *tert*-butylmethyl ether, dimethyl sulfoxide (DMSO), ethanol, ethyl acetate, ethyl ether, ethyl formate, formic acid, heptane, isobutyl acetate, isopropyl acetate, methyl acetate, 3-methyl-1-butanol, methylethyl ketone, 2-methyl-1-propanol, pentane, 1-pentanol, 1-propanol, propyl acetate, trimethylamine, dimethyl formamide, and any combinations or mixtures thereof. Any one of these solvents may be used for dissolving the one or more PACA (homo)polymers and/or one or more PACA copolymers according to the methods of the present invention. In various embodiments, the solvent is selected from any of acetone, ethanol, acetic acid, formic acid, ethyl acetate, dimethyl sulfoxide (DMSO), dimethyl formamide, butyl acetate, isobutyl acetate, and any combinations or mixtures thereof. In particularly preferred embodiments, the solvent is acetone. Such embodiments have been shown to generate average fiber diameters of about 2 µm, using a polymer solution of about 1%. In various other preferred embodiments, the solvent is an acetone:ethanol mixture. Preferably, the solvent is an acetone:ethanol mixture of about 4:1 (v/v). Such embodiments have been shown to generate average fiber diameters of about 700±100 nm, using a polymer solution of about 1%.

The following solvents, for which according to the U.S. FDA no toxicological data were found, may also be used in the context of the present disclosure: any one of 1,1-diethoxypropane, 1,1-dimethoxymethane, 2,2-dimethoxypropane, isooctane, isopropylether, methyl isopropylketone, methyltetrahydrofuran, petroleumether, trichloroacetic acid, and trifluoro acetic acid. In various preferred embodiments, the solvent is trichloroacetic acid or trifluoroacetic acid, or a mixture thereof. In various preferred embodiments, the solvent is a mixture of acetone:trichloroacetic acid, or acetone:trifluoroacetic acid. Such mixtures may be used in order to prepare blend solutions of PACA and non-PACA polymers as described herein, in particular blend solutions of PACA and chitosan, or PACA and PCL. Preferably, the mixture of acetone:trichloroacetic acid, or acetone:trifluoroacetic acid, is a mixture of about 4:1 (v/v).

In accordance with the above, the PACA-based nano/microfibers or PACA-based nano/microfiber meshes provided by the present invention may be considered biocompatible accordingly, in particular biocompatible with skin or tissue. The terms "skin" or "tissue" as used herein encompass animal and human skin or tissue. The PACA-based nano/microfibers or PACA-based nano/microfiber meshes may be considered biocompatible because they are non-toxic and/or do not have any deleterious effect on the human or animal body, and can therefore be employed in (therapeutic) biomedical applications and/or drug products. Advantageously, the methods in accordance with the present invention provide PACA-based nano/microfibers or PACA-based nano/microfiber meshes, which as such are ready to use, *e.g*., no further manufacturing steps are necessary to obtain a product ready for application. Accordingly, the ready-to-use nano/microfibers or ready-to-use nano/microfiber meshes in accordance with the present invention preferably do not comprise or need a support. Advantageously, biodegradable nano/microfibers or nano/microfiber meshes can be used for various different biomedical applications, including therapeutic biomedical applications, and can be degraded over time without the need for a support. In particular, biodegradable nano/microfibers or nano/microfiber meshes of the present invention can advantageously be used in dental applications, including in particular dental surgery like, *e.g*., oral maxillofacial surgery. Furthermore, there are no concerns regarding the compatibility of the material of the support with the material(s) of the nano/microfibers or the nano/microfiber meshes. In addition, when used for medical applications, there are no concerns regarding any tolerance issues of the support material vis-à-vis a patient to be treated. Thus, in various embodiments, the PACA-based nano/microfibers or PACA-based nano/microfiber meshes produced are ready-to-use PACA-based nano/microfibers or ready-to-use PACA-based nano/microfiber meshes, respectively, because the nano/microfibers are not comprised by a support. In particular, in various embodiments, after removing the PACA-based nano/microfibers or PACA-based nano/microfiber meshes from the collector to obtain ready-to-use PACA-based nano/microfibers or PACA-based nano/microfiber meshes, respectively, the methods for producing ready-to-use PACA-based nano/microfibers, or ready-to-use PACA-based nano/microfiber meshes, do not comprise a subsequent step of providing the PACA-based nano/microfibers or PACA-based nano/microfiber meshes on a support.

In the present invention, the **(alkyl) cyanoacrylate component** includes at least one (alkyl) cyanoacrylate monomer such as those represented by the **formula H₂C=C(CN)-COOR,** wherein R includes, without being limited thereto, any C₁₋₁₅ alkyl groups. The alkyl chain may be a straight chain or a branched chain C₁₋₁₅ alkyl chain. Specifically, the at least one cyanoacrylate monomer of the present disclosure may be represented by the formula H₂C=C(CN)-COOR, wherein R may be any of a C₁ alkyl chain, a C₂ alkyl chain, a C₃ alkyl chain, a C₄ alkyl chain, a C₅ alkyl chain, a C₆ alkyl chain, a C₇ alkyl chain, a C₈ alkyl chain, a C₉ alkyl chain, a C₁₀ alkyl chain, a C₁₁ alkyl chain, a C₁₂ alkyl chain, a C₁₃ alkyl chain, a C₁₄ alkyl chain, a C₁₅ alkyl chain, and mixtures thereof. Preferably, the alkyl chain is a straight chain or a branched chain C₁₋₁₀ alkyl chain, more preferably the alkyl chain is a straight chain or a branched chain C₁₋₈ alkyl chain.

Accordingly, in various aspects of the present invention, the alkyl substituent of the ACA monomer has a chain length from 1 to 15 carbon atoms, preferably a chain length from 1 to 10 carbon atoms, more preferably a chain length from 1 to 8 carbon atoms. In various preferred embodiments, the alkyl chain is a straight C₁₋₁₀ alkyl chain or a branched C₁₋₁₀ alkyl chain. More preferably, the alkyl chain is a straight C₁₋₈ alkyl chain or a branched C₁₋₈ alkyl chain. Still more preferably, the alkyl chain is a straight C₁₋₄ alkyl chain or a branched C₁₋₄ alkyl chain. Even more preferably, the alkyl chain is a straight C₁₋₃ alkyl chain or a branched C₁₋₃ alkyl chain. In particularly preferred embodiments, the alkyl chain is a straight C₁₋₂ alkyl chain or a branched C₁₋₂ alkyl chain. Desirably, the straight alkyl chain is a **linear** straight alkyl chain.

In accordance with the above, in various aspects of the present invention, the alkyl substituent of each monomer unit making up an ACA oligomer as described herein has a chain length from 1 to 15 carbon atoms, preferably a chain length from 1 to 10 carbon atoms. Preferably, the alkyl chain of each monomer unit making up the at least one or more ACA oligomers is a straight C₁₋₁₀ alkyl chain or a branched C₁₋₁₀ alkyl chain. More preferably, the alkyl chain of each monomer unit making up the at least one or more ACA oligomers is a straight C₁₋₈ alkyl chain or a branched C₁₋₈ alkyl chain. Still more preferably, the alkyl chain of each monomer unit making up the at least one or more ACA oligomers is a straight C₁₋₅ alkyl chain or a branched C₁₋₅ alkyl chain. Even more preferably, the alkyl chain of each monomer unit making up the at least one or more ACA oligomers is a straight C₁₋₃ alkyl chain or a branched C₁₋₃ alkyl chain. In particularly preferred embodiments, the alkyl chain of each monomer unit making up the at least one or more ACA oligomers is a straight C₁₋₂ alkyl chain or a branched C₁₋₂ alkyl chain. Desirably, the straight alkyl chain of each monomer unit making up the at least one or more ACA oligomers is a **linear** straight alkyl chain.

Branched alkanes may be derived from the (linear) straight chain alkanes system by removing one of the hydrogen atoms from a methylene group (-CH2-) and replacing it with an alkyl group (the alkyl group thus being the substituent). Accordingly, in various embodiments of the present disclosure, one or more methylene groups (-CH2-) of a branched alkyl chain disclosed herein are replaced with an alkyl group substituent. Thus, in various embodiments, a branched alkyl chain described herein is characterized by an alkyl group substituent being attached to the alkyl chain. The alkyl group substituent attached to the alkyl chain of the at least one cyanoacrylate monomer may be any of a C₁₋₁₅ alkyl group. Preferably, the alkyl group substituent attached to the alkyl chain of the at least one cyanoacrylate monomer is any of a C₁₋₁₀ alkyl group. More preferably, the alkyl group attached to the alkyl chain of the at least one cyanoacrylate monomer is any of a C₁₋₈ alkyl group. Even more preferably, the alkyl group substituent attached to the alkyl chain of the at least one cyanoacrylate monomer is any of a C₁₋₄ alkyl group.

Likewise, the alkyl group substituent attached to the alkyl chain of each monomer unit making up the at least one or more ACA oligomers may be any of a C₁₋₁₅ alkyl group. Preferably, the alkyl group substituent attached to the alkyl chain of each monomer unit making up the at least one or more ACA oligomers is any of a C₁₋₁₀ alkyl group. More preferably, the alkyl group substituent attached to the alkyl chain of each monomer unit making up the at least one or more ACA oligomers is any of a C₁₋₈ alkyl group, and still more preferably any of a C₁₋₄ alkyl group. The alkyl group substituents may include heteroatoms and functional groups such as a hydroxy group, a substituent including an ether linkage, an amino group, a carboxy group, and mixtures thereof. A more preferred substituent including heteroatoms is illustrated by monomer butyl-lactoyl-2-cyanoacrylate (BLCA). In one preferred embodiment, the substituent only comprises carbon and hydrogen atoms.

The alkyl group attached to the alkyl chain of the at least one cyanoacrylate monomer can be a straight chain or a branched chain alkyl group. Preferably, the alkyl group attached to the alkyl chain of the at least one cyanoacrylate monomer is a straight chain alkyl group. Likewise, in various embodiments of the present disclosure, the alkyl chain of the at least one cyanoacrylate monomer is a straight alkyl chain.

In line with the foregoing explanations, in the present disclosure, poly(alkyl cyanoacrylates) that are produced by polymerization may be considered as (linear) straight chain type, or branched chain type poly(alkyl cyanoacrylate) polymers.

In various embodiments, the (alkyl) cyanoacrylate monomer or monomer unit is selected from at least one of methyl cyanoacrylate, 2-methoxy ethyl cyanoacrylate, ethyl cyanoacrylate, propyl cyanoacrylate, butyl cyanoacrylate (such as *n*-butyl 2-cyanoacrylate), hexyl cyanoacrylate, iso hexyl (2-)cyanoacrylate, octyl cyanoacrylate, (2-)octyl cyanoacrylate, isobutyl (2-)cyanoacrylate, and combinations thereof. In preferred embodiments, the (alkyl) cyanoacrylate monomer or monomer unit is selected from at least one of methyl cyanoacrylate, ethyl cyanoacrylate, butyl cyanoacrylate (such as *n*-butyl 2-cyanoacrylate), octyl cyanoacrylate, and combinations thereof. In various other preferred embodiments, the (alkyl) cyanoacrylate monomer or monomer unit is selected from at least one of methyl cyanoacrylate, ethyl cyanoacrylate, octyl cyanoacrylate, and combinations thereof. In still other preferred embodiments, the (alkyl) cyanoacrylate monomer is selected from at least one of methyl cyanoacrylate, ethyl cyanoacrylate, butyl cyanoacrylate, and combinations thereof. In various further embodiments, the cyanoacrylate monomer is selected from ethyl cyanoacrylate, butyl cyanoacrylate and combinations thereof. A particularly desirable cyanoacrylate monomer or monomer unit includes ethyl cyanoacrylate. Suitably, the cyanoacrylate monomer or monomer unit may be ethyl cyanoacrylate. Another particularly preferred cyanoacrylate monomer or monomer unit is butyl cyanoacrylate (such as *n*-butyl 2-cyanoacrylate). The aforementioned ACA monomers or monomer units may form the basis of ACA oligomers as described herein. Thus, in various embodiments of the present invention, the one or more ACA oligomers are selected from oligomers of any of the aforementioned ACA monomers or monomer units.

In various embodiments of the methods for producing (ready-to-use) PACA-based nano/microfibers or PACA-based nano/microfiber meshes, the one or more ACA monomers, or the at least two different ACA monomer species, are selected from any of n-butyl-2-cyanoacrylate (BCA), iso-butyl-2-cyanoacrylate (IBCA), n-pentyl-2-cyanoacrylate (PCA), isopentyl-2-cyanoacrylate (IPCA), n-hexyl-2-cyanoacrylate (HCA), iso-hexyl-2-cyanoacrylate (IHCA), cyclo-hexyl-2-cyanoacrylate (CHCA), n-heptyl-2-cyanoacrylate (HepCA), iso-heptyl-2-cyanoacrylate (IHepCA), n-octyl-2-cyanoacrylate (OCA), iso-octyl-2-cyanoacrylate (IOCA), butyl-lactoyl-2-cyanoacrylate (BLCA), and any combinations or mixtures thereof. The aforementioned one or more ACA monomers may form the basis of ACA oligomers as described herein. Thus, in various embodiments of the present invention, the one or more ACA oligomers are selected from oligomers of any of the aforementioned one or more ACA monomers. Accordingly, in various embodiments of the methods for producing (ready-to-use) PACA-based nano/microfibers or PACA-based nano/microfiber meshes, the one or more ACA oligomers are selected from ACA oligomers of any of n-butyl-2-cyanoacrylate (BCA), oligomers of iso-butyl-2-cyanoacrylate (IBCA), oligomers of n-pentyl-2-cyanoacrylate (PCA), oligomers of iso-pentyl-2-cyanoacrylate (IPCA), oligomers of n-hexyl-2-cyanoacrylate (HCA), oligomers of iso-hexyl-2-cyanoacrylate (IHCA), oligomers of cyclo-hexyl-2-cyanoacrylate (CHCA), oligomers of n-heptyl-2-cyanoacrylate (HepCA), oligomers of iso-heptyl-2-cyanoacrylate (IHepCA), oligomers of n-octyl-2-cyanoacrylate (OCA), oligomers of iso-octyl-2-cyanoacrylate (IOCA), oligomers of butyl-lactoyl-2-cyanoacrylate (BLCA), and any combinations or mixtures thereof.

In a separate aspect, the present disclosure encompasses the use of allyl cyanoacrylates and/or aryl cyanoacrylates in the methods and products as described herein. Accordingly, in one aspect, the method described herein can be performed with allyl cyanoacrylates and poly(allyl cyanoacrylate) polymers, respectively, in order to produce ready-to-use poly(allyl cyanoacrylate) based nano/microfibers instead of poly(alkyl cyanoacrylate) based nano/microfibers. The method steps of such an allyl cyanoacrylate-based method are analogous to the method steps of the method of producing ready-to-use poly(alkyl cyanoacrylate) based nano/microfibers described herein. Consequently, the present disclosure encompasses allyl cyanoacrylate-related and poly(allyl cyanoacrylate)polymer-related aspects and embodiments analogous to any corresponding alkyl cyanoacrylate-related and poly(alkyl cyanoacrylate)polymer-related aspects and embodiments described herein. Such allyl cyanoacrylate-related and poly(allyl cyanoacrylate)polymer-related aspects and embodiments encompass in particular poly(allyl cyanoacrylate) based nano/microfibers that can be obtained by the corresponding method of producing ready-to-use poly(allyl cyanoacrylate) based nano/microfibers, as well as products and uses that can be generated or are otherwise derived or based on such obtained poly(allyl cyanoacrylate) based nano/microfibers.

Likewise, in one aspect the method described herein can be performed with aryl cyanoacrylates and poly(aryl cyanoacrylate) polymers, respectively, in order to produce ready-to-use poly(aryl cyanoacrylate) based nano/microfibers instead of poly(alkyl cyanoacrylate) based nano/microfibers. The method steps of such an aryl cyanoacrylate-based method are analogous to the method steps of the method of producing ready-to-use poly(alkyl cyanoacrylate) based nano/microfibers described herein. Consequently, the present disclosure encompasses aryl cyanoacrylate-related and poly(aryl cyanoacrylate)polymer-related aspects and embodiments analogous to any corresponding alkyl cyanoacrylate-related and poly(alkyl cyanoacrylate)polymer-related aspects and embodiments described herein. Such aryl cyanoacrylate-related and poly(aryl cyanoacrylate)polymer-related aspects and embodiments encompass in particular poly(aryl cyanoacrylate) based nano/microfibers that can be obtained by the corresponding method of producing ready-to-use poly(aryl cyanoacrylate) based nano/microfibers, as well as products and uses that can be generated or are otherwise derived or based on such obtained poly(aryl cyanoacrylate) based nano/microfibers.

As described herein, the terms "alkyl cyanoacrylate" and "cyanoacrylate" may be used interchangeably. The alkyl substituent of the "alkyl cyanoacrylate" may be unsubstituted or may optionally be substituted, as explained in more detail elsewhere herein. Substituents may include heteroatoms and functional groups such as hydroxy groups, a substituent including an ether linkage, a carboxy group, and any combinations or mixtures thereof. A more preferred substituent including heteroatoms is illustrated by the monomer butyl-lactoyl-2-cyanoacrylate (BLCA). In preferred embodiments, the substituent only comprises carbon and hydrogen atoms.

In various embodiments, the methods for producing (ready-to-use) PACA-based nano/microfibers or PACA-based nano/microfiber meshes comprise in step (a) a **single PACA-homopolymer**, which is accordingly made up of one single ACA monomer species, or one single ACA oligomer species. In other words, such methods do not comprise any further PACA-homopolymer species. In such embodiments, the PACA-based nano/microfibers or PACA-based nano/microfiber meshes produced by the methods of the invention are made up of one single PACA-homopolymer, *i.e*., do not comprise any further PACA-homopolymer species. Furthermore, such embodiments are characterized by a polydispersity index, which is due to the single PACA-homopolymer. As described elsewhere herein, the polydispersity index encompasses a ratio of the weight-average molecular weight (M_{w}) to the number-average molecular weight (Mₙ) of the PACA homopolymer from 1 to 1.7. In various embodiments, the polydispersity index is based on one specific number-average molecular weight (Mₙ), which may be any of 10³ to 10⁶ Da. In other words, in various embodiments, the single PACA homopolymer species, which is made up of one single ACA monomer (or one single ACA oligomer), has **one specific** number-average molecular weight (**Mₙ**), which may be any of 10³ to 10⁶ Da. Preferably, the one specific number-average molecular weight (Mₙ) may be any of 10⁴ to 10⁶ Da, preferably 10⁴ Da or 10⁶ Da. In various preferred embodiments, the one specific number-average molecular weight (Mₙ) is 10⁵ or 10⁶ Da. In other embodiments, the polydispersity index is based on **different** number-average molecular weights (**Mₙ**), which may be any of 10³ to 10⁶ Da. In other words, in various embodiments, the single PACA homopolymer species, which is made up of one single ACA monomer (or one single ACA oligomer), has different number-average molecular weights (Mₙ), which may be any of 10³ to 10⁶ Da. Preferably, the different number-average molecular weights (Mₙ) may be any of 10⁴ to 10⁶ Da, preferably 10⁴ Da or 10⁶ Da. In various preferred embodiments, the different number-average molecular weights (Mₙ) are 10⁵ and 10⁶ Da. In various further embodiments, the single PACA homopolymer species, which is made up of one single ACA monomer (or one single ACA oligomer species), has different number-average molecular weights (Mₙ) in different proportions, wherein the polydispersity index of the single PACA homopolymer species is from 1 to 1.7.

In various other embodiments, the methods for producing (ready-to-use) PACA-based nano/microfibers or PACA-based nano/microfiber meshes comprise in step (a) **at least two PACA homopolymer species**, which are made up of different ACA monomer species, or different ACA oligomer species. In such embodiments, the PACA-based nano/microfibers or PACA-based nano/microfiber meshes produced by the methods of the invention are made up of at least two PACA homopolymer species made up of different ACA monomer species (or different ACA oligomer species). Furthermore, such embodiments are characterized by a polydispersity index, which is due to the at least two PACA-homopolymer species. The polydispersity index encompasses a ratio of the weight-average molecular weight (M_{w}) to the number-average molecular weight (Mₙ) of the **at least two PACA homopolymer species** from 1 to 1.7. The polydispersity index is based on different number-average molecular weights (Mₙ) of the at least two PACA homopolymer species, which may be any of 10³ to 10⁶ Da. Preferably, the number-average molecular weights (Mₙ) may be any of 10⁴ to 10⁶ Da, preferably 10⁴ Da or 10⁶ Da. In various preferred embodiments, the different number-average molecular weights (Mₙ) are 10⁵ and 10⁶ Da. On the other hand, also encompassed are embodiments, in which the polydispersity index of the at least two PACA-homopolymer species is based on one specific number-average molecular weight (Mₙ), which may be any of 10³ to 10⁶ Da. In other words, in such embodiments, all of the at least two PACA homopolymer species, which are made up of different ACA monomers (or different ACA oligomers), have the same specific number-average molecular weight (Mₙ), which may be any of 10³ to 10⁶ Da. Preferably, the number-average molecular weights (Mₙ) may be any of 10⁴ to 10⁶ Da, preferably 10⁴ Da or 10⁶ Da. In various preferred embodiments, the different number-average molecular weights (Mₙ) are 10⁵ and 10⁶ Da. In various embodiments, the polydispersity index of the at least two PACA homopolymer species made up of different ACA monomer species (or different ACA oligomer species) is based on different number-average molecular weight (Mₙ) in different proportions, wherein the polydispersity index of the at least two PACA homopolymer species is from 1 to 1.7.

In preferred embodiments of the above-described methods for producing (ready-to-use) PACA-based nano/microfibers or PACA-based nano/microfiber meshes comprising in step (a) at least two PACA homopolymer species, the methods comprise in step (a) any of two, three, four and five PACA homopolymer species, which are made up of different ACA monomer species, or different ACA oligomer species. More preferably, the methods comprise in step (a) two or three PACA homopolymer species, which are made up of different ACA monomer species, or different ACA oligomer species.

In various embodiments, the methods for producing (ready-to-use) PACA-based nano/microfibers or PACA-based nano/microfiber meshes comprise in step (a) a **single PACA copolymer**, which is made up of at least two different ACA monomer species, or at least two different ACA oligomer species. In other words, such methods do not comprise any further PACA copolymer species. In such embodiments, the PACA-based nano/microfibers or PACA-based nano/microfiber meshes produced by the methods of the invention are made up of one single PACA copolymer, *i.e*., do not comprise any further PACA copolymer species. In preferred embodiments, the single PACA copolymer is made up of any of two, three, four, and/or five different ACA monomer species, or is made up of any of two, three, four, and/or five different ACA oligomer species. More preferably, the single PACA copolymer is made up of two or three different ACA monomer species, or is made up of two or three different ACA oligomer species.

Furthermore, such embodiments are characterized by a polydispersity index, which is due to the single PACA copolymer. As described elsewhere herein, the polydispersity index encompasses a ratio of the weight-average molecular weight (M_{w}) to the number-average molecular weight (Mₙ) of the PACA copolymer from 1 to 1.7. In various embodiments, the polydispersity index is based on one specific number-average molecular weight (Mₙ), which may be any of 10³ to 10⁶ Da. In other words, in various embodiments, the single PACA copolymer species, which is made up of at least two different ACA monomer species, or at least two different ACA oligomer species, has **one specific** number-average molecular weight (**Mₙ**), which may be any of 10³ to 10⁶ Da. Preferably, the one specific number-average molecular weight (Mₙ) may be any of 10⁴ to 10⁶ Da, preferably 10⁴ Da or 10⁶ Da. In various preferred embodiments, the one specific number-average molecular weight (Mₙ) is 10⁵ or 10⁶ Da. In other embodiments, the polydispersity index is based on **different** number-average molecular weights (**Mₙ**), which may be any of 10³ to 10⁶ Da. In other words, in various embodiments, the single PACA copolymer species, which is made up of at least two different ACA monomer species, or at least two different ACA oligomer species, has different number-average molecular weights (Mₙ), which may be any of 10³ to 10⁶ Da. Preferably, the different number-average molecular weights (Mₙ) may be any of 10⁴ to 10⁶ Da, preferably 10⁴ Da or 10⁶ Da. In various preferred embodiments, the different number-average molecular weights (Mₙ) are 10⁵ and 10⁶ Da. In various further embodiments, the single PACA copolymer species, which is made up of at least two different ACA monomer species, or at least two different ACA oligomer species, has different number-average molecular weights (Mₙ) in different proportions, wherein the polydispersity index of the single PACA copolymer species is from 1 to 1.7.

In various other embodiments, the methods for producing (ready-to-use) PACA-based nano/microfibers or PACA-based nano/microfiber meshes comprise in step (a) **at least two PACA copolymer species**, which are made up of different ACA monomer species, or different ACA oligomer species. In such embodiments, the PACA-based nano/microfibers or PACA-based nano/microfiber meshes produced by the methods of the invention are made up of at least two PACA copolymer species made up of different ACA monomer species, or different ACA oligomer species. Furthermore, such embodiments are characterized by a polydispersity index, which is due to the at least two PACA copolymer species. The polydispersity index encompasses a ratio of the weight-average molecular weight (M_{w}) to the number-average molecular weight (Mₙ) of the **at least two PACA copolymer species** from 1 to 1.7. The polydispersity index is based on different number-average molecular weights (Mₙ) of the at least two PACA copolymer species, which may be any of 10³ to 10⁶ Da. Preferably, the number-average molecular weights (Mₙ) may be any of 10⁴ to 10⁶ Da, preferably 10⁴ Da or 10⁶ Da. In various preferred embodiments, the different number-average molecular weights (Mₙ) are 10⁵ and 10⁶ Da. On the other hand, also encompassed are embodiments, in which the polydispersity index of the at least two PACA copolymer species is based on one specific number-average molecular weight (Mₙ), which may be any of 10³ to 10⁶ Da. In other words, in such embodiments, all of the at least two PACA copolymer species, which are made up of different ACA monomer species, or different ACA oligomer species, have the same specific number-average molecular weight (Mₙ), which may be any of 10³ to 10⁶ Da. Preferably, the number-average molecular weights (Mₙ) may be any of 10⁴ to 10⁶ Da, preferably 10⁴ Da or 10⁶ Da. In various preferred embodiments, the different number-average molecular weights (Mₙ) are 10⁵ and 10⁶ Da. In various embodiments, the polydispersity index of the at least two PACA copolymer species made up of different ACA monomer species (or different ACA oligomer species) is based on different number-average molecular weight (Mₙ) in different proportions, wherein the polydispersity index of the at least two PACA copolymer species is from 1 to 1.7. In preferred embodiments of the above-described methods for producing (ready-to-use) PACA-based nano/microfibers or PACA-based nano/microfiber meshes comprising in step (a) at least two PACA copolymer species, the methods comprise in step (a) any of two, three, four and five PACA copolymer species, which are made up of different ACA monomer species, or different ACA oligomer species. More preferably, the methods comprise in step (a) two or three PACA copolymer species, which are made up of different ACA monomer species, or different ACA oligomer species.

As described herein, an ACA oligomer may be composed of any of two to ten ACA monomer units, including dimers trimers, tetramers, pentamers, hexamers, hepatmers, octamers, nonamers and decamers accordingly.

PACA polymers, preferably high molecular weight PACA (homo)polymers (*e.g*., PBCA), as used in the present invention may be produced by anionic bulk or mass polymerization. High molecular weight PACA (homo)polymers may be characterized by a molecular weight in the range of 10⁴ to 10⁶ Da. Preferably, the said molecular weight denotes the number-average molecular weight. DMPT may be used as initiator in anionic polymerization, in particular anionic bulk or mass polymerization. In various embodiments of the present invention, the anionic polymerization as disclosed herein is performed in the presence of an initiator or catalyst. In various embodiments, the initiator or catalyst is an anionic initiator or anionic catalyst. In various embodiments, the initiator or catalyst is a tertiary amine, preferably an aromatic amine. In various preferred embodiments, the initiator or catalyst is *N,N*'-dimethyl-*p*-toluidine (DMPT). In various embodiments, the initiator is dissolved in a solvent (1/1000, v/v). Preferably, the solvent is acetone. The anionic (bulk or mass) polymerization may be carried out by first adding ACA to a reaction chamber (e.g., a flask, more specifically a three-neck round-bottom flask), followed by addition of the initiator. Preferably, the polymerization reaction is performed in a cold reaction system (e.g., the polymerization reaction may be performed over a cold water bath). At the end of the polymerization, the solid formed may be solved, precipitated in distilled water, filtrated, washed with water first and subsequently with methanol, and dried in vacuum. The following Table 1 may exemplify the identification of PACAs with the number-average molecular weight values and the molar relation between ACA and initiator.

**Table 1. Experimental conditions and results of BCA bulk anionic polymerization.**

| PBCA | BCA:DMPT | **M̅ₙ̅** [Da] | **M̅_̅{̅w̅}̅/M̅ₙ̅** |
|---|---|---|---|
| AM | 4.10³ :1 | 1.1·10^{6*}; 8.0·10³ | 2.7; 2.7 |
| IAM | 2.10³ :1 | 2.5·10⁵ | 4.5 |
| IIAM | 2-10² :1 | 7.4·10⁴ | 1.7 |
| IIIAM | 1.10³ :1 | 1.2·10⁴ | 1.4 |

| | | | |
|---|---|---|---|
| *polymeric population in higher proportion. AM, IAM, IIAM, IIIAM: designation of different polymers. | | | |

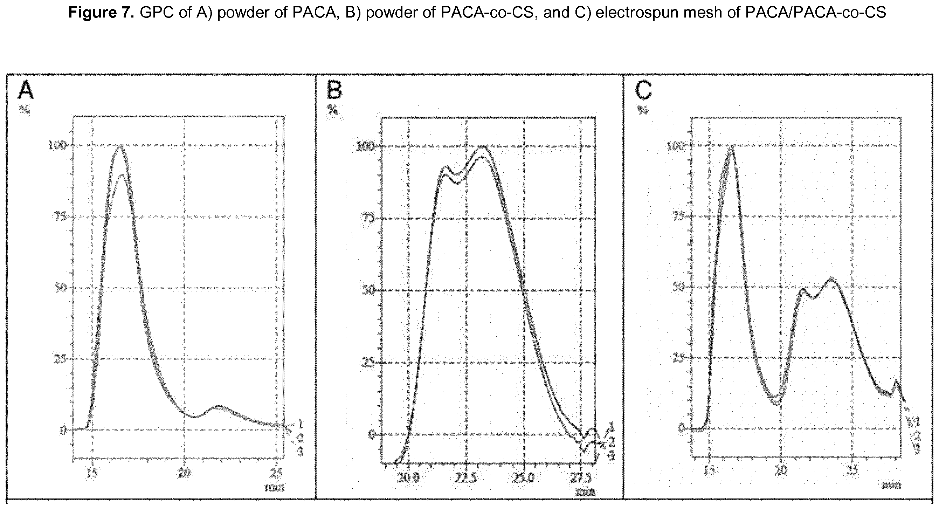

Bulk polymerization or mass polymerization may be carried out by adding a soluble (radical) initiator to pure monomer in liquid state. The initiator should dissolve in the monomer. The reaction is initiated by heating or exposing to radiation. As the reaction proceeds the mixture becomes more viscous. The reaction is exothermic and a wide range of molecular masses are produced. In various embodiments of the present invention, the anionic polymerization of ACAs is performed with in bulk (*i.e*., pure ACA monomer in liquid state). In various other embodiments of the present invention, the anionic polymerization is performed with ACAs produced from ACA oligomers, which result from Knoevenagel condensation of alkyl cyanoacetate and formaldehyde, followed by pyrolysis of the oligomeric mixture formed. PACAs can be obtained via a direct Knoevenagel condensation between the corresponding alkyl cyanoacetate and formaldehyde, or by anionic polymerization of their monomers. Figure 7 shows the GPC results of A) powder of PACA, B) powder of PACA-co-CS, and C) electro-spun mesh of PACA/PACA-co-CS.

The polymerization of ACAs using an anionic initiator, preferably a tertiary amine, more preferably DMPT, has been successfully performed in a wide range of polymer/initiator ratios. In various embodiments of the present invention, the ACA and the initiator/catalyst may be applied to the anionic polymerization reaction in a specific ratio. For example, in various embodiments, the ratio of ACA:initiator/catalyst may be about 1·10³:1. In various other embodiments, the ratio of ACA:initiator/catalyst may be about 2·10²:1. These ratios allow the production of PACAs with a number-average molecular weight between 10⁴ to 10⁶ Da. The number-average molecular weight may be defined as the total weight (mass) of polymer divided by the total number of molecules (arithmetic mean of the molar mass distribution). The weight-average molecular weight relates to the sum of all molecular weights (sum of the products of the molar mass of each fraction) multiplied by their weight fraction. The polydispersity index gives a measure of the distribution of the molecular weight within a sample. It has a value greater than or equal to one: it is equal to one only if all the molecules have the same weight (*i.e*., if it is monodisperse), and the further away it is, the larger the spread of molecular weights. In the method of the present invention, well-defined polymeric materials are used rather than a polymer mixture or copolymer mixture comprising vastly differing molecular weights.

In various preferred embodiments of the present invention, the ACA is BCA and the initiator is a tertiary amine, preferably the initiator is DMPT.

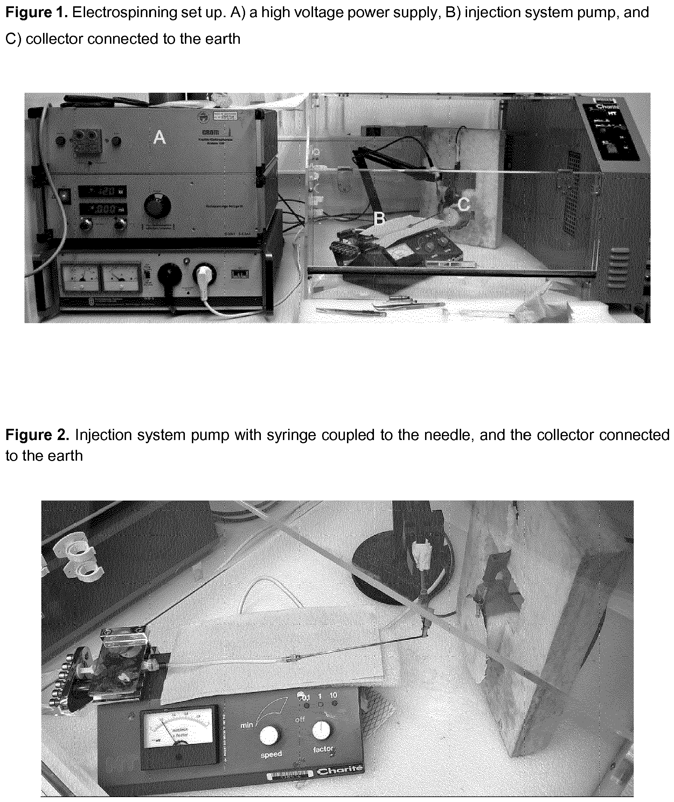

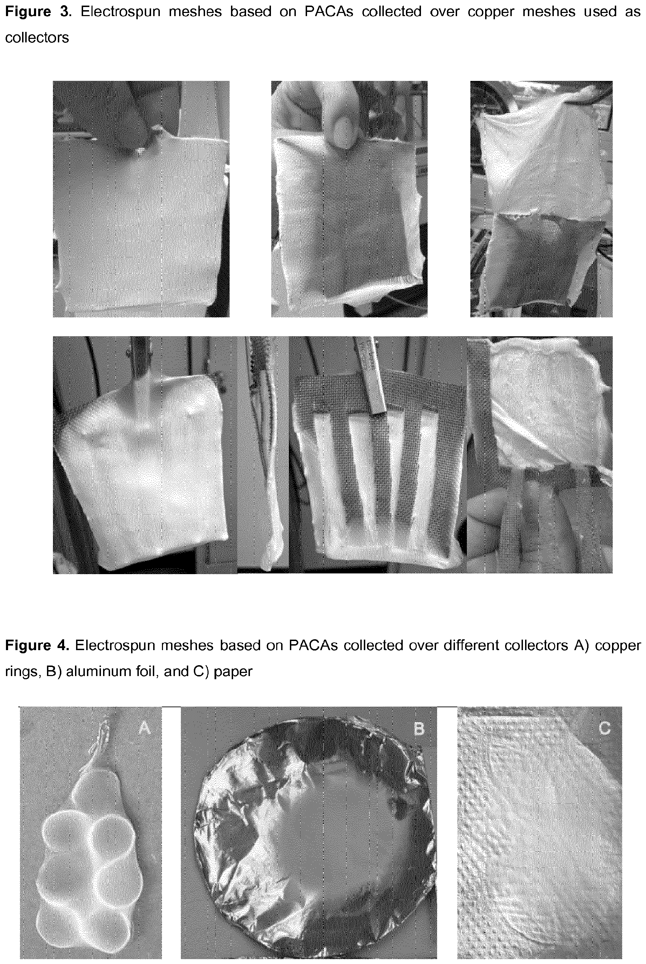

In various embodiments, the electrospinning parameters according to the present disclosure may be any one of the following: voltage = 5-30 kV, preferably 5-22 kV; fluid rate = 0.025 to 2.000 mL/min, preferably 0.025-0.125 mL/min; needle tip diameter = 0.3-1 mm; needle tip-collector distance = 5-30 cm, preferably 5-15 cm; polymer concentration = 0.5-50% w/w; and/or solvent mixture = acetone:ethanol from 1:0 to 4:1 (v/v). During the step of electrospinning, a polymer solution jet (or a polymer melt) is charged with an electrical potential that breaks the surface tension of solution drops at the end of a needle tip due to the repulsion of the same charge. This induces the charged jet movement to a collector connected to an opposite power or to the earth. In the most basic form, an electrospinning machine consists of a high voltage power supply, an injection system pump, a syringe with a needle, and a collector. The shape of the drops in the moment of break is named "Taylor cone" (the conical surface of the solution at the needle tip). Next, the charged jet is dried during the itinerary to the collector and stretched when solvent is evaporated, forming very thin fibers in the nanometer to micrometer range. Figure 1 illustrates a general setup for electrospinning: A) a high voltage power supply, B) an injection system pump, and C) a collector connected to the earth. Figure 2 illustrates an injection system pump with the syringe coupled to the needle, and the collector connected to the earth. Generally, typical collectors suitable for the method in accordance with the present invention include copper meshes, copper rings, aluminum foil, paper, and stainless steel squares, without being limited thereto. Figure 3 shows electro-spun meshes based on PACAs collected over copper meshes used as collectors. Figure 4 shows electro-spun meshes based on PACAs collected over different collectors including A) copper rings, B) aluminum foil, and C) paper.

It has been surprisingly found that a solution comprising (i) a PACA homopolymer and/or a PACA copolymer, and (ii) a non-PACA polymer (which may be a non-PACA homopolymer and/or a non-PACA copolymer) can be successfully applied in electrospinning for producing ready-to-use PACA-based nano/microfibers and/or PACA-based nano/microfiber meshes. Therefore, in various embodiments, the methods for producing (ready-to-use) PACA-based nano/microfibers and/or PACA-based nano/microfiber meshes comprise in step (b) the addition of at least one non-PACA polymer (which may be a non-PACA homopolymer and/or a non-PACA copolymer) to the solution obtained in step (b) prior to electrospinning, *i.e*., the solution obtained by dissolving the PACA homopolymer and/or PACA copolymer of step (a) in a solvent. In various embodiments, the non-PACA polymer (which may be a non-PACA homopolymer and/or a non-PACA copolymer) may be any of poly(lactic-co-glycolic acid) (PLGA), poly(ε-caprolactone) (PCL), poly(ethyelenglycol) (PEG), poly(lactic acid) (PLA), chitosan (CH), hyaluronic acid (HA), dextran (Dex), and any combination or mixtures thereof, without being limited thereto. In various embodiments, the non-PACA copolymer is poly(lactic-co-glycolic acid) (PLGA) or poly(lactic acid) (PLA). In various preferred embodiments, the non-PACA homopolymer is poly(lactic acid) (PLA). In various other preferred embodiments, the non-PACA homopolymer is **chitosan or PLGA**.

In various other embodiments, the non-PACA polymer (which may be a non-PACA homopolymer and/or a non-PACA copolymer) may be a bioadhesive non-PACA polymer (including a bioadhesive non-PACA homopolymer and/or bioadhesive non-PACA copolymer). As described herein, bioadhesion refers to the ability of certain synthetic and biological macromolecules to adhere to biological tissues. Bioadhesion may depend in part upon the properties of the polymer, biological tissue, and the surrounding environment. Several factors have been found to contribute to a polymer's bioadhesive capacity: the presence of functional groups able to form hydrogen bridges (-OH, -COOH), sufficient elasticity for the polymeric chains to interpenetrate the mucous layer, and high molecular weight. Accordingly, in various embodiments, the bioadhesive non-PACA polymer is characterized by the presence of one or more functional groups, which are able to form hydrogen bridges, *e.g*., -OH, -COOH. Bioadhesion systems have been used in dentistry, orthopedics, ophthalmology, and in surgical applications.

The amount of the non-PACA polymer (which may be a non-PACA homopolymer and/or a non-PACA copolymer) added to the solution comprising a PACA homopolymer and/or a PACA copolymer can vary widely, typically falling in the range of about 0.1 % and about 75% by weight based on the weight of the solution comprising (i) a PACA homopolymer and/or a PACA copolymer, and (ii) a non-PACA polymer (which may be a non-PACA homopolymer and/or a non-PACA copolymer). In some embodiments, the concentration of the non-PACA polymer (which may be a non-PACA homopolymer and/or a non-PACA copolymer) falls in the range of about 0.5-5% by weight; in some embodiments, the concentration of the non-PACA polymer (which may be a non-PACA homopolymer and/or a non-PACA copolymer) falls in the range of about 0.7-4% by weight; in some embodiments, the concentration of the non-PACA polymer (which may be a non-PACA homopolymer and/or a non-PACA copolymer) falls in the range of about 1-3% by weight. In various other embodiments, the concentration of the non-PACA polymer (which may be a non-PACA homopolymer and/or a non-PACA copolymer) falls in the range of about 25-75% by weight. Preferably, the concentration of the non-PACA polymer (which may be a non-PACA homopolymer and/or a non-PACA copolymer) falls in the range of about 25-75% by weight if the non-PACA homopolymer is PLGA, more preferably if the non-PACA homopolymer is PLGA and the PACA is PBCA.

in some embodiments, the concentration of the non-PACA polymer (which may be a non-PACA homopolymer and/or a non-PACA copolymer) falls in the range of about 0.7-4% by weight; in some embodiments, the concentration of the non-PACA polymer (which may be a non-PACA homopolymer and/or a non-PACA copolymer) falls in the range of about 1-3% by weight.

As described elsewhere herein, the poly(alkyl cyanoacrylate) copolymer in step (a) of the methods described herein may comprises (i) at least two different ACA monomer species, (ii) at least two alkyl cyanoacrylate oligomers comprising at least two different ACA monomer species, or (iii) at least one poly(alkyl cyanoacrylate) (PACA) polymer and at least one non-PACA polymer. Accordingly, with regard to the aforementioned (iii), the PACA copolymer may be obtainable by, or may be obtained, or may be generated by, ionic polymerization of one or more ACA monomers with one or more non-PACA polymers. Such non-PACA polymers may catalyze or initiate the ionic polymerization of the ACA monomers by virtue of ionic groups of the non-PACA polymers, *i.e*., the ionic groups serve as the initiator of the anionic polymerization of the ACA monomers. In various embodiments, the aforementioned ionic polymerization is anionic polymerization, or is cationic polymerization. Preferably, ionic polymerization is anionic polymerization.

In various embodiments of any of the methods disclosed herein, a non-PACA polymer as used in the present invention is capable of initiating anionic polymerization of ACA monomers. A non-PACA polymer as used in the present invention, specifically a non-PACA polymer being capable of initiating anionic polymerization of ACA monomers, may have an anionic group (*i.e*., may be an anionic non-PACA polymer), or may have a cationic group (*i.e*., may be a cationic non-PACA polymer), or may also be a neutral non-PACA polymer. In various preferred embodiments, a non-PACA polymer as used in the present invention may be an organic non-PACA polymer, preferably an organic anionic, or an organic cationic non-PACA polymer, *i.e*., may have an organic anionic group, or may have an organic cationic group, respectively. The disclosure also encompasses organic neutral non-PACA polymers. As described herein, an anionic group may be considered as a group that carries an overall negative charge. Likewise, a cationic group may be considered as a group that carries an overall positive charge. Furthermore, a neutral non-PACA polymer may be considered as a non-PACA polymer with an overall neutral charge. Furthermore, any anionic non-PACA polymer as described herein may be considered as anionic non-PACA polymer with an overall negative charge. Likewise, any cationic non-PACA polymer as described herein may be considered as cationic non-PACA polymer with an overall positive charge.

In preferred embodiments, the (organic) anionic group of an anionic non-PACA polymer described herein may be an amine group, or a hydroxyl group, or a carboxyl group. In various embodiments, a non-PACA polymer as described herein may be a polysaccharide, a glycosaminoglycan, or a polyether. In preferred embodiments, a non-PACA polymer as described herein is a polysaccharide. The polysaccharide may be considered as a neutral, cationic, or anionic polysaccharide. Likewise, the glycosaminoglycan and polyether may be considered as a neutral, cationic, or anionic glycosaminoglycan or polyether. In more preferred embodiments, a non-PACA polymer as described herein may be any of polyethylene glycol (PEG), chitosan, dextran, and hyaluronic acid. In particularly preferred embodiments, the non-PACA polymer is chitosan. In preferred embodiments, any of the non-PACA polymers as described herein is a biodegradable non-PACA polymer. The term "biodegradable non-PACA polymer" may be considered as a non-PACA polymer, which is degradable by biological activity. More specifically, such biological activity may be enzymatic hydrolysis, or decomposition by microorganisms, in particular by bacteria. The decomposition by microorganisms, in particular by bacteria, may include enzymatic hydrolysis.

As further described herein, any of the PACA (homo)polymers disclosed herein may also be considered biodegradable PACA (homo)polymers. The term "biodegradable PACA polymer" may be considered as a PACA (homo)polymer, which is degradable by biological activity. More specifically, such biological activity may be enzymatic hydrolysis, or decomposition by microorganisms, in particular by bacteria. The decomposition by microorganisms, in particular by bacteria, may include enzymatic hydrolysis.

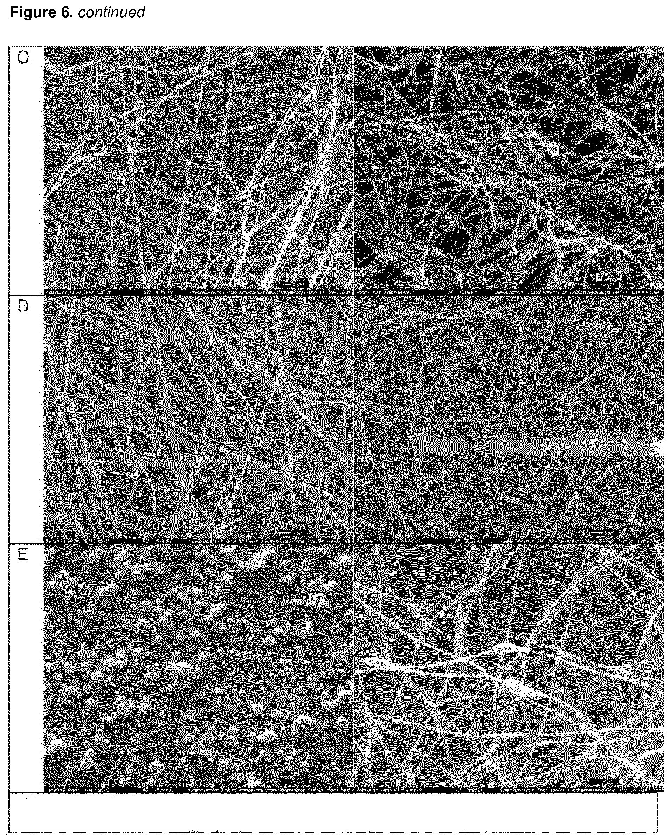

Chitosan is produced by deacetylation of chitin, which is the structural component in the exoskeleton of crustaceans and cell walls of fungi. Chitosan is a polymer composed of randomly distributed *β*-(1,4)-linked D-glucosamine (deacetylated unit) and *N*-acetly-D-glucosamine (acetylated unit). Chitin is a useful biocompatible material that can be used in medical applications including, but not limited to, tissue regeneration and tissue engineering. Chitosan materials contemplated for use herein can be further characterized by the percentage of deacetylation of said chitosan, which in some embodiments can be up to 100%; in some embodiments, the chitosan has a percentage of deacetylation from about 65 up to about 100%; in some embodiments, the chitosan has a percentage of deacetylation from about 70 up to about 100%; in some embodiments, the chitosan has a percentage of deacetylation from about 75 to about 100%; in some embodiments, the chitosan has a percentage of deacetylation from about 80 up to about 100%. In some embodiments, chitosan contemplated for use herein may be of any source of animal or microbial origin; in some embodiments, chitosan contemplated for use herein is of crustacean origin. As readily recognized by those of skill in the art, the amount of chitosan present in the formulations contemplated herein can vary widely, typically falling in the range of about 0.1% and about 30% by weight based on the weight of the solution comprising (i) a PACA homopolymer and/or a PACA copolymer, and (ii) chitosan, or comprising (i) a PACA (homo)polymer and (ii) a non-PACA polymer, respectively. In some embodiments, the chitosan concentration falls in the range of about 0.5-5% by weight; in some embodiments, the chitosan concentration falls in the range of about 0.7-4% by weight; in some embodiments, the chitosan concentration falls in the range of about 1-3% by weight. In various preferred embodiments of the present disclosure, the weight ratio of chitosan:PACA is about 3·10³:1, *e.g*., the chitosan weight is 0.3%. Thus, in various preferred embodiments, the chitosan concentration is about 0.3% by weight. In the electrospun mesh shown in Fig. 6E, right, the weight ratio between PACA and PACA-co-chitosan was 50:50, *i.e*., the weight of the chitosan was 0.15% relative to the total PACA polymer. Thus, in various preferred embodiments, the chitosan concentration is about 0.15% by weight. In various other preferred embodiments, the chitosan concentration falls in the range of about 0.15% to about 0.3% by weight.

Chitosan materials contemplated for use herein can be further characterized with reference to one or more of the following: a moisture content of < 15%; an ash content <1%, a turbidity of <50 NTU, a solubility of >99%, and/or <3 ppm heavy metals (*i.e*., As, Cd, Pb, Hg). Chitosan-containing ready-to-use PACA-based nano/microfibers and/or PACA-based nano/microfiber meshes contemplated herein can be used in a variety of ways, *e.g*., for the targeted treatment of wounds, aches and pains, skin inflammation due to insect bites, bedsores, ulcers, and the like. In some embodiments of the present invention, chitosan-containing ready-to-use PACA-based nano/microfibers and/or PACA-based nano/microfiber meshes contemplated herein may be suitable for uses such as cosmetic and/or personal care applications to: (i) cover and protect as a film-forming ingredient to favor moisture control; (ii) provide antimicrobial activity as a natural preservative; and/or (iii) act as a carrier of other active ingredients for slow release, due to ionic bonding to compounds and chitosan degradation by natural skin enzymes.

As described herein, the present disclosure encompasses PACA-based nano/microfibers and/or PACA-based nano/microfiber meshes that are obtainable by, obtained by, or generated by, any of the methods of the present invention. Such PACA-based nano/microfibers and/or PACA-based nano/microfiber meshes are novel over the art because of the structural characteristics conferred on the PACA-based nano/microfibers and/or PACA-based nano/microfiber meshes of the present invention by virtue of the steps and materials (i.e., ACA monomers, and/or ACA oligomers, and/or PACA homopolymers, and/or PACA copolymer) used in the methods provided by the present invention. In particular, the ratio of the weight-average molecular weight (M_{w}) to the number-average molecular weight (Mₙ) of the poly(alkyl cyanoacrylate) homopolymer and/or poly(alkyl cyanoacrylate) copolymer, which (polydispersity) ratio can be from 1 to 1.7, provides for a coining feature on the novel PACA-based nano/microfibers and/or PACA-based nano/microfiber meshes provided by the present invention. As described herein, the PACA-based nano/microfibers and/or PACA-based nano/microfiber meshes provided by the present invention may be characterized by any of the structural features that are described in relation to the PACA-based nano/microfibers and/or PACA-based nano/microfiber meshes and/or ACA monomers and ACA oligomers referred to in the methods of the present disclosure.

In some aspects, the disclosure provides methods of promoting oral health (*e.g*., promoting gum health), comprising orally applying (or administering) to a subject any of the chitosan-containing ready-to-use PACA-based nano/microfibers disclosed herein to the soft or hard tissues of the mouth. In an analogous embodiment, the disclosure provides any of the chitosan-containing ready-to-use PACA-based nano/microfibers disclosed herein for use in promoting oral health (*e.g*., promoting gum health). In another analogous embodiment, the disclosure provides uses of any of the chitosan-containing ready-to-use PACA-based nano/microfibers disclosed herein for the manufacture of a medical device or medicament for promoting oral health (*e.g*., promoting gum health). The disclosure provides methods of treating disorders of the oral mucosa and/or the tooth supporting tissues, such as treating peri-implantitis, comprising orally applying or administering to a subject any of the chitosan-containing ready-to-use PACA-based nano/microfibers disclosed herein.

As described already elsewhere herein, the ratio of the weight-average molecular weight (M_{w}) to the number-average molecular weight (Mₙ) of the poly(alkyl cyanoacrylate) homopolymer and/or poly(alkyl cyanoacrylate) copolymer is from 1 to 1.7. Accordingly, this ratio is considered to describe the **polydispersity index** of the of the poly(alkyl cyanoacrylate) homopolymer and/or poly(alkyl cyanoacrylate) copolymer. Preferably, the ratio of the weight-average molecular weight (M_{w}) to the number-average molecular weight (Mₙ) is from 1.1 to 1.5. More preferably, the ratio of the weight-average molecular weight (M_{w}) to the number-average molecular weight (Mₙ) is from 1.2 to 1.4. The molecular weight may be determined using gel permeation chromatography (GPC). The determination may be based on polystyrene or PMMA as a standard using either DMF, acetone, or THF as solvents. The measurements may be carried out under ambient temperature and pressure, *e.g*., 25°C and 1 ATM.

The present disclosure provides PACA-based nano/microfibers or PACA-based nano/microfiber meshes prepared or obtainable by any of the methods described herein. Advantageously, the PACA-based nano/microfibers or PACA-based nano/microfiber meshes prepared or obtainable by any of the methods described herein may be considered as ready-to-use PACA-based nano/microfibers or ready-to-use PACA-based nano/microfiber meshes. In various aspects, the PACA-based nanofibers of the present invention may have **an (average) diameter** in the range from about 1 nm to about 999 nm, which includes (average) diameters in the range between 0.01 nm to 1 nm. In various embodiments, the PACA-based nanofibers of the present invention have an (average) diameter in the range from about 100 nm to about 900 nm. In various other embodiments, the PACA-based nanofibers of the present invention have an (average) diameter in the range from about 200 nm to about 800 nm. In further embodiments, the PACA-based nanofibers of the present invention have an (average) diameter in the range from about 300 nm to about 700 nm. In still other embodiments, the PACA-based nanofibers of the present invention have an (average) diameter in the range from about 400 nm to about 600 nm.

In various other embodiments, the PACA-based nanofibers of the present invention have an (average) diameter in the range from about 600 nm to about 990 nm. In still other embodiments, the PACA-based nanofibers of the present invention have an (average) diameter ≤ about 600 nm, preferably in the range from about 200 nm to about 600 nm, or in the range from about 100 nm to about 600 nm, more preferably in the range from about 200 nm to about 500 nm, or in the range from about 100 nm to about 500 nm. It is considered that mechanical tensile properties are further improved with decreasing fiber diameter according to the results comparing PACA meshes with decreasing fiber diameters.

In further preferred embodiments, the PACA-based nano/microfibers of the present invention have an (average) diameter of about 1,000 ± 300 nm, more preferably of about 970 ± 280 nm, or of about 800 ± 300 nm, more preferably of about 780 ± 300 nm. In various other preferred embodiments, the PACA-based nano/microfibers of the present invention have an (average) diameter of about 1,000 ± 200 nm, more preferably of about 1,000 ± 200 nm, or of about 800 ± 200 nm, more preferably of about 780 ± 200 nm.

Likewise, the PACA-based microfibers of the present invention may have an average diameter in the micrometer size range. In various aspects, PACA-based microfibers of the present invention may have an (average) diameter in the range from about 1 µm to about 10 µm, which includes (average) diameters in the range between 0.01 µm to 1 µm. In various embodiments, the PACA-based microfibers of the present invention may have an (average) diameter in the range from about 1 µm to about 5 µm, preferably in the range of about 1 µm to about 4 µm, more preferably in the range of about 1 µm to about 3 µm, still more preferably in the range of about 1 µm to about 2 µm. In various other embodiments, the PACA-based microfibers of the present invention may have an (average) diameter in the range from about 2 µm to about 5 µm, preferably in the range from about 2 µm to about 4 µm. In further embodiments, the PACA-based microfibers of the present invention may have an (average) diameter in the range from about 3 µm to about 5 µm, preferably in the range from about 3 µm to about 4 µm.

In various embodiments, the fiber average diameter of PACA-based nano/microfibers or the nano/microfibers of the PACA-based nano/microfiber meshes may be from 1 nm to 500 µm. Preferably, the fiber average diameter of PACA-based nano/microfibers or the nano/microfibers of the PACA-based nano/microfiber meshes may be from 10 nm to 50 µm. More preferably, the fiber average diameter of PACA-based nano/microfibers or the nano/microfibers of the PACA-based nano/microfiber meshes may be from 100 nm to 5 µm. In various embodiments, the novel PACA-based nano/microfibers and/or the PACA-based nano/microfiber meshes of the present invention may be **modified chemically and/or physically for biomedical applications**, preferably wherein the surface of the novel PACA-based nano/microfibers and the PACA-based nano/microfiber meshes of the present invention is modified by encapsulation and/or immobilization of a therapeutic agent and/or biological material. In particular, in various embodiments, the novel PACA-based nano/microfibers and/or the PACA-based nano/microfiber meshes of the present invention may be immobilized with, or have encapsulated, or intersperse, at least one of a therapeutic agent, antibiotic, antiviral agent, chemotherapeutic agent, analgesic and analgesic combinations, anti-inflammatory agent, vitamin, sedative, radiopharmaceutical, metal particles, metal alloy particles, metal oxide particles, hydroxyapatite, sodium alginate, stains, cells, protein, peptides, nucleic acids, nucleic acid analogs, nucleotides or oligonucleotides, peptide nucleic acids, aptamers, antibodies or fragments or portions thereof, antigens or epitopes, hormones, hormone antagonists, growth factors or recombinant growth factors and fragments and variants thereof, cell attachment mediators, cytokines, enzymes, and/or any combination or mixture thereof. The therapeutic agent can, for example, be dissolved in the solution to be electro-spun, can alternatively be co-electrospun, or used for coaxially electro-spinning into the center of the fibers. They can be distributed homogeneously in the body of the nano/microfiber(s) and/or mesh(es) using simultaneous electrospinning of the PACA solution and electrospraying of the therapeutic agent solution, or can be loaded in microparticles distributed homogeneously in the PACA-based nano/microfiber(s) and/or meshes by electrospinning of one emulsion comprising the PACA(s) and therapeutic agent-loaded microparticles, or can be coated onto the surface of the mesh(es) by adsorption or chemical bonding. The way to incorporate the therapeutic agent into the nano/microfiber(s) and/or mesh(es) and its quantity will depend on the properties of the agent and the biomedical application to be performed.

The meshes in accordance with the present invention are suitable for various applications, including biomedical products for wound dressing/healing, tissue regeneration/engineering, membrane for cell separating, organ protection, implant coating, controlled drug delivery and *in vitro* cell culture. Preferred applications include dental applications, in particular applications in dental surgery.

In various embodiments, the novel PACA-based nano/microfibers and/or the PACA-based nano/microfiber meshes of the present invention may include living cells seeded into/onto the meshes or nano/microfibers. The cells can be any type of animal cell, such as human cells, or even cells from the subject to receive the treatment. In various embodiments, the novel PACA-based nano/microfibers and/or the PACA-based nano/microfiber meshes of the present invention may comprise a cell growth medium.

The encapsulation of any active agent or a combination of active agents allows using meshes for numerous biomedical purposes. The ready-to-use nano/microfiber meshes of the present disclosure are suitable for the delivery of cargo molecules such as pharmaceutically or cosmetically active agents and nutritional supplements (herein also generally referred to as "active agents"). In various embodiments, the active agent is soluble in the same solvent as the PACA or PACA-based nano/microfiber mesh. This allows for fibers or meshes where the active agent is completely homogeneous in the PACA matrix, although this is not obligatory because there are dissimilar strategies to encapsulate any type of active agents (hydrophobic and hydrophilic) using electro-spinning, for example preparing a solvent mixture able to solve both the polymers and active agents, or preparing emulsions composed by (1) the polymer which will produce the fiber, and (2) polymeric particles which charge the active agent; it is also possible to make coaxial or triaxial electrospinning, etc. On the other hand, active agents can be physically or chemically immobilized over the ready electro-spun meshes.

The ready-to-use nano/microfiber meshes of the present disclosure can increase the bioavailability and efficacy of the immobilized or encapsulated active agent(s) by protecting said agent(s) from premature degradation, and allowing for a sustained release thereof. The ready-to-use nano/microfiber meshes of the present invention may have an advantageously high load of any cargo molecules, (therapeutic/active) agents and/or biological material. Thus, the ready-to-use nano/microfiber meshes of the invention can comprise at least 50 wt-%, at least 60 wt-%, at least 70 wt-%, preferably at least 80 wt-%, more preferably at least 90 wt-%, at least 95 wt-%, most preferably at least 99 wt-% or at least 99.9 wt-% and up to 99.9 wt-%, up to 99.95 wt-% or, preferably, up to 99.99 wt-% of any active agent(s), therapeutic agent(s) and/or biological material, relative to the total weight of poly(alkyl cyanoacrylate) polymers and active agent(s), therapeutic agent(s) and/or biological material of a ready-to-use nano/microfiber mesh.

The present invention provides nano/microfiber meshes or nano/microfiber networks comprising the novel PACA-based nano/microfibers disclosed herein. As described herein, the terms "nano/microfiber mesh" and "nano/microfiber network" may be used interchangeably. The present invention also provides composite materials comprising (i) PACA-based nano/microfibers or PACA-based nano/microfiber meshes disclosed herein, and (ii) a non-PACA polymer (which may be a non-PACA homopolymer and/or a non-PACA copolymer). The nature and embodiments of such non-PACA polymers (which may be a non-PACA homopolymers and/or a non-PACA copolymers) is described elsewhere herein and encompassed as embodiments by the composite materials provided by the present invention. In various preferred embodiments, the composite material comprises (i) PACA-based nano/microfibers or PACA-based nano/microfiber meshes disclosed herein, and (ii) chitosan or PLGA.

As explained herein above, the present invention demonstrates that meshes or mesh networks of PACA nano/microfibers can be produced by electrospinning of PACA solutions. Different types of meshes have been produced, as shown in the Examples and the accompanying drawings. In particular, it is shown, e.g., that meshes can be produced based on PACA alone, in particular based on PBCA (poly (n-butyl cyanoacrylate)) alone.

PACAs with different molecular weights can be used for electrospinning, from high molecular weight (10⁶ Da) to low molecular weight (10³ Da). Preferably, the said molecular weight denotes the number-average molecular weight. In various embodiments of the present invention, the PACA polymer concentration, in particular the PBCA concentration, is between 0.5 to 50% w/w, preferably in acetone or acetone/ethanol mixtures. In various embodiments, the PACA is a PACA homopolymer, in particular a PBCA homopolymer, with a **high molecular weight**, e.g., 10⁵ Da or 10⁶ Da, and the polymer concentration (PACA, in particular PBCA) is in the range between 0.5 to 15% w/w in the solution. In various preferred embodiments, the solvent is acetone or an acetone/ethanol mixture. Preferably, the polymer concentration (PACA, in particular PBCA) of the high molecular weight polymer is in the range between 0.5 to 10% w/w, wherein the solvent preferably is acetone or an acetone/ethanol mixture. More preferably, the polymer concentration (PACA, in particular PBCA) of the high molecular weight polymer is in the range between 0.5 to 5% w/w, wherein the solvent preferably is acetone or an acetone/ethanol mixture. Still more preferably, the polymer concentration (PACA, in particular PBCA) of the high molecular weight polymer is in the range between 0.5 to 2% w/w or between 0.5 to 1.5% w/w, wherein the solvent preferably is acetone or an acetone/ethanol mixture. In particularly preferred embodiments, the high molecular weight polymer (PACA, in particular PBCA) has a concentration of about 0.5% w/w, wherein the solvent preferably is acetone or an acetone/ethanol mixture. Such embodiments can result in the formation of PACA nanofibers (in particular PBCA nanofibers) having an average fiber diameter in the nanometer and micrometer range. For example, meshes produced using a PACA (in particular PBCA) polymer concentration of 0.5% w/w (the solvent preferably being acetone or an acetone/ethanol mixture) comprise PACA nanofibers (in particular PBCA nanofibers) having an average fiber diameter of about 600 nm. Such meshes are encompassed by the disclosure of the present invention. Furthermore, meshes produced using a PACA (in particular PBCA) polymer concentration of 1.1% w/w (the solvent preferably being acetone or an acetone/ethanol mixture) comprise PACA nanofibers (in particular PBCA nanofibers) having an average fiber diameter of about 1.1 µm. Such meshes are also encompassed by the disclosure of the present invention. Thus, the fiber diameter of the meshes increases with the concentration of the polymer solution. In various embodiments of the present invention, the PACA polymer concentration may have a polymer concentration from 0.1% w/w to about 1.0% w/w (the solvent preferably being acetone or an acetone/ethanol mixture), when aiming at producing PACA-based fibers having an average fiber diameter in the nanometer range. In various other embodiments of the present invention, the PACA polymer concentration may have a polymer concentration ≥1.1% w/w (the solvent preferably being acetone or an acetone/ethanol mixture), when aiming at producing PACA-based fibers having an average fiber diameter in the micrometer range. The thickness of meshes as described in this paragraph is around 260 µm. Furthermore, the Young's modulus of these meshes may be around 1.2 MPa, while the tensile strength may be about 0.08 MPa, and the failure strain may be about 15%. Thus, the mechanical properties of meshes provided by the present invention are closer to elastic materials than rigid materials.

It is surprisingly demonstrated that continuous and smooth fibers can be obtained using very low viscous (0.3 - 3 cP) PACA solutions (in particular PBCA solutions) as described in the preceding paragraph, something that is, to the best of the inventors' knowledge, unknown or not possible using other polymers like PCL. Accordingly, in various embodiments of the present invention, the PACA solutions (in particular PBCA solutions) disclosed herein exhibit a viscosity below 10 cP, preferably below 5 cP, more preferably below 3 cP. In various preferred embodiments of the present invention, the PACA solutions (in particular PBCA solutions) disclosed herein exhibit a viscosity in the range between 0.3 to 3 cP.

In various embodiments, meshes that are based on a blend solution of a high molecular weight PACA polymer, preferably a high molecular weight PACA homopolymer (*e.g*., PBCA), and a non-PACA polymer, preferably a non-PACA homopolymer (*e.g*., chitosan), may have a thickness of about 400 µm with an average fiber diameter of about 1 µm, a Young's modulus of about 1 MPa, a tensile strength of about 0.07 MPa, and 14% of failure strain. Such meshes may furthermore have a minimum pore area of 0.1 µm², and a maximum pore area of about 482 µm².

In various other embodiments, meshes that are based on a blend solution of a high molecular weight PACA polymer, preferably a high molecular weight PACA homopolymer (*e.g*., PBCA), and a non-PACA polymer, preferably a non-PACA copolymer (*e.g*., PLGA) may have a thickness of about 390 µm with an average fiber diameter of about 900 nm, a Young's modulus of about 1 MPa, a tensile strength of about 0.06 MPa, and 20% of failure strain. Such meshes may furthermore have a pore area in the range of 0.1 µm² to 160 µm².

In various other embodiments, meshes that are based on a solution of a high molecular weight PACA polymer, preferably a high molecular weight PACA homopolymer (*e.g*., PBCA), and a therapeutic agent, *e.g*., 5FU, may have a thickness of about 400 µm with an average fiber diameter in the range between 700 nm to 1.2 µm, a Young's modulus of about 0.7 MPa, a tensile strength of about 0.1 MPa, and 25% of failure strain. The average fiber diameter increases (from 700 nm to 1.2 µm) with increasing drug concentration: 7, 10, 15, 20, 25 and 30% relative to the mass of PACA polymer, wherein the PACA polymer preferably is a high molecular weight PACA (homo)polymer. Preferably, such a high molecular weight PACA (homo)polymer is dissolved in a solvent at a concentration from 1- 2% w/w. Preferably, the solvent is acetone.

In line with the above, in various embodiments of the present invention, a nano/microfiber mesh as disclosed herein, preferably meshes prepared based on a high molecular weight PACA (homo)polymer), has a thickness in the micrometer range, preferably below 700 µm, more preferably below 500 µm, still more preferably below 400 µm, and even more preferably below 300 µm. In various embodiments, the thickness is ≤ 260 µm. In various embodiments of the present invention, the pore area of the meshes disclosed herein is in the range of 0.1-370 µm². In various embodiments, the average fiber diameter of such meshes having a thickness in the micrometer range, preferably below 700 µm, more preferably below 500 µm, still more preferably below 400 µm, and even more preferably below 300 µm, may be in the range between 500 nm to 1.5 µm. In various further embodiments, such meshes may furthermore have a Young's modulus in the range of about 0.5 MPa to 1.5 MPa. In still further embodiments, such meshes may furthermore have a tensile strength in the range of about 0.05 MPa to 0.15 MPa. In various preferred embodiments, the average fiber diameter of meshes as disclosed herein, preferably meshes prepared based on a high molecular weight PACA (homo)polymer, and having a thickness in the micrometer range, preferably below 700 µm, more preferably below 500 µm, still more preferably below 400 µm, and even more preferably below 300 µm, may be in the range between 500 nm to 1.5 µm, and the meshes may furthermore have a Young's modulus in the range of about 0.5 MPa to 1.5 MPa, a tensile strength in the range of about 0.05 MPa to 0.15 MPa. Furthermore, such meshes may have about 15%-25% of failure strain. In even more preferred embodiments, meshes as disclosed herein, preferably meshes prepared based on a high molecular weight PACA (homo)polymer), may have a thickness in the range ≤ 400 µm, with the average fiber diameter of such meshes being in the range between 500 nm and 1.5 µm, preferably in the range between 500 nm to 1.5 µm. More preferably, such meshes may furthermore have a Young's modulus in the range of about 0.5 MPa to 1.5 MPa, preferably in the range of about 0.7 MPa to 1.2 MPa. In still more preferred embodiments, such meshes may furthermore have a tensile strength in the range of about 0.05 MPa to 0.15 MPa, preferably in the range of about 0.06 MPa to 0.1 MPa. Furthermore, such meshes may have about 15%-25% of failure strain.

Table 2 shows physical and mechanical properties of electro-spun meshes encompassed by the present disclosure.

| **Table 2.** Physical and mechanical properties of electro-spun meshes | | | | | | |
|---|---|---|---|---|---|---|
| Sample name | Contact angle | Φ_{fiber} (µm) | Porosity (%) | Thickness (µm) | Young's modulus | Tensile strength |
| PACA | 147 | 0.4-2.0 | 40-70 | 120-700 | 0.3-3.0 | 0.02-0.2 |
| PACA/PLGA | 148 | 0.8-2.0 | 30-50 | 200-400 | 0.2-4.0 | 0.02-0.2 |
| PLGA | 153 | 0.1-1.0 | 40-60 | 180-400 | 0.3-4.0 | 0.06-0.3 |
| PACA/PACA-co-CS | 134 | 0.8-1.0 | 60-80 | 300-500 | 0.4-2.0 | 0.04-0.1 |
| PACA/5FU | - | 0.2-2.0 | 20-70 | 200-400 | 0.2-0.7 | 0.03-0.2 |

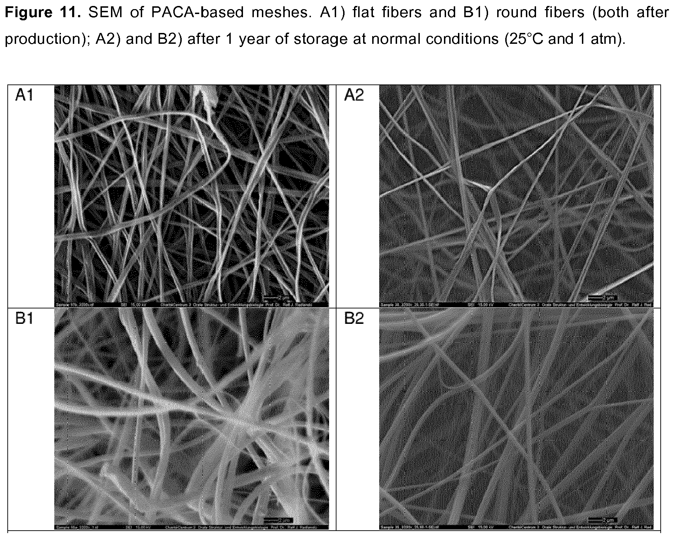

The tensile properties of meshes composed of PACAs with number average molecular weights in the order of 10⁶ Da may be as follows: Young's modulus = 0.3-3 MPa, and tensile strength = 0.02-0.2 MPa. These meshes are stable at least for 23 days in culture medium with human gingival fibroblasts. Figure 11 is a SEM picture of a PBCA mesh before and after 23 days in incubation with FHGFib.

In various embodiments of the present disclosure, meshes as disclosed herein, preferably nano/microfiber meshes prepared based on a high molecular weight PACA (homo)polymer) as described in the preceding paragraphs, may have a pore area in the range of 0.1 µm² to 500 µm². Preferably, such meshes may be based on electrospinning of a blend solution of a high molecular weight PACA polymer, preferably a high molecular weight PACA homopolymer (*e.g*., PBCA), and a non-PACA polymer, preferably a non-PACA homo- or copolymer (*e.g*., chitosan or PLGA, respectively). In various other embodiments of the present disclosure, meshes as disclosed herein, preferably nano/microfiber meshes prepared based on a high molecular weight PACA (homo)polymer) as described in the preceding paragraphs, may have a pore area in the range of 0.1 µm² to 370 µm², wherein the meshes may be based on electrospinning of a solution not containing a non-PACA polymer or a therapeutic agent, but only a high molecular weight PACA polymer, preferably a high molecular weight PACA homopolymer (*e.g*., PBCA).

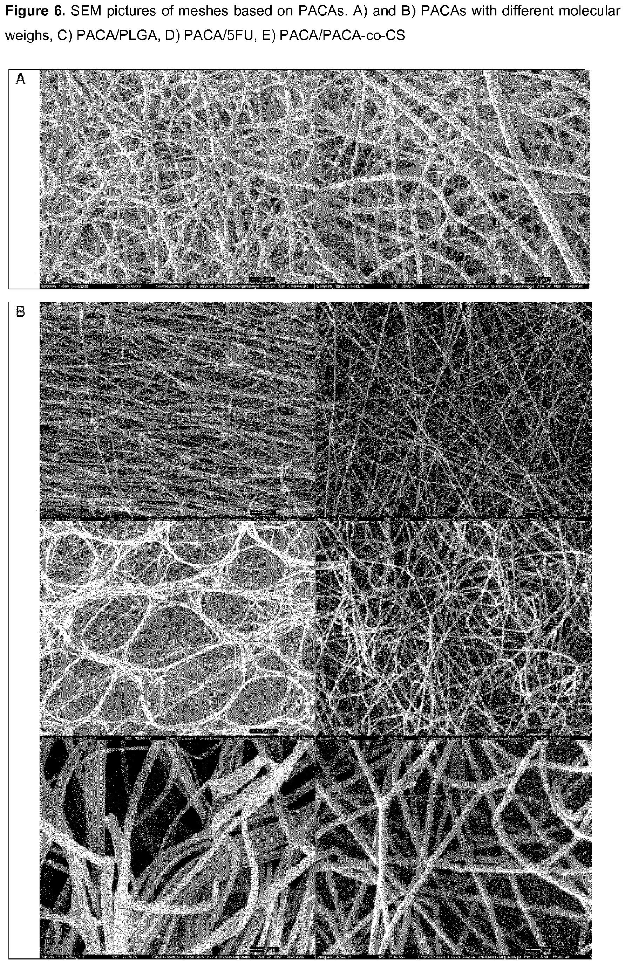

As illustrated herein, multilayered electro-spun biodegradable hydrophobic meshes composed of PACA/PLGA random nano/microfibers are obtained, with good adhesion between nanofiber layers, and porous morphology. These meshes can serve for cell attachment and proliferation and favor tissue repair by its own hemostatic and bacteriostatic properties. Meshes based on PACA/PLGA can be composed of a mixture of ribbon like nanofibers and full cylinder like nanofibers (illustrated in Figure 6). The thickness of these meshes may generally be below 500 µm. The average fiber diameter may range from 800 nm to 2 µm. The porosity of the meshes may be between 30% and 50%. Their mechanical properties may be as follows: Young's modulus = 0.2-4.0 MPa, and tensile strength = 0.02-0.2 MPa.

In addition, multilayered electro-spun biodegradable meshes composed of PACAs and chitosan random nano/microfibers are obtained, with good adhesion between nanofiber layers, and porous morphology. These meshes can be employed for cell attachment and proliferation and favors tissue repair by its own hemostatic and bacteriostatic properties. PACA/PACA-co-CS-50% meshes composed of a mixture of ribbon like nanofibers and full cylinder like nanofibers may have average fiber diameters around 1 µm, and porosity of about 70%. The thickness of these meshes may be below 500 pm. Their mechanical properties may be as follows: Young's modulus = 0.8 ± 0.3 MPa, and tensile strength = 0.06 ± 0.02 MPa. Electrospinning of PACA-based solutions - any polymeric solution containing PACA - can be employed to produce meshed for potential biomedical applications. PACAs with different number-average molecular weights (Mn) were synthesized (10⁶ Da, 10⁵ Da, 10⁴ Da, 10³ Da) using bulk anionic polymerization of the corresponding monomer with anionic initiators with a weight-average molecular weight in the range of 10⁶-10⁴ Da.

Multilayered electro-spun biodegradable meshes composed of PACAs random nano/microfibers, charged or immobilized with, or having encapsulated, therapeutic agents/other chemical substances, or with particles of one biodegradable polymer charged with these therapeutic agents/other chemical substances, with good adhesion between nanofiber layers, and porous morphology can be obtained. These meshes can be employed for cell attachment and proliferation, favor tissue repair by its own hemostatic and bacteriostatic properties and may be used to deliver therapeutic agents/other chemical substances. Meshes were composed of homogeneous nano/microfibers (see Figure 6) where PACAs and the other component were homogeneously mixed in a common solvent and electro-spun. The thickness of these meshes was below 500 µm with average fiber diameters ranging from 200 nm to 2 µm and a porosity between 20 and 70%. The mechanical properties of one of the formulations (PBCA/5FU_15%) were: Young's modulus = 0.4 ± 0.2 MPa, and tensile strength = 0.08 ± 0.04 MPa.

The novel PACA-based nano/microfiber meshes of the present invention may, for example, be provided in square shape, such as 10x10 cm. Said squares may then be cut in the shape considered more adequate for the intended application. It is advantageously not necessary to apply the meshes to an adhesive or support because the PACA-based nano/microfiber meshes of the present invention are adhesive to the skin by themselves. Nevertheless, if deemed to be advantageous, an adhesive may be applied to further improve the adhesiveness, for example a cyanoacrylate adhesive, so as to adhere the edges of the mesh to the place of application.

The **porosity** of the novel PACA-based nano/microfiber meshes of the present invention is preferably between 40 and 70%, more preferably between 50 and 70%. The meshes are effective as a protective barrier, which inhibit, *e.g*., exogenous microorganism invasions into wounds. In contrast to films formed of PACAs, which are generally non-porous materials, the meshes are characterized by being highly porous with well-interconnected pores, which, for example, allow exuding fluid from wounds, allow gas permeation and allow cell proliferation and migration. The fibrous meshes of the present invention are advantageous over films of the same PACA materials because they have a larger surface area for cell attachment and cell proliferation. The porous meshes offer a high surface-to-volume ratio, may assist the control of fluid drainage, and may serve as delivery platform for therapeutic agents, other chemical substances or biological material, as described elsewhere herein. Porosity can generally be determined by porosimetry, by measuring the apparent material density, BET analysis or by microscope images. Porosity may be determined using BET analysis with nitrogen as the adsorbate. Porosity values can also be obtained by analysis of SEM pictures using ImageJ software.

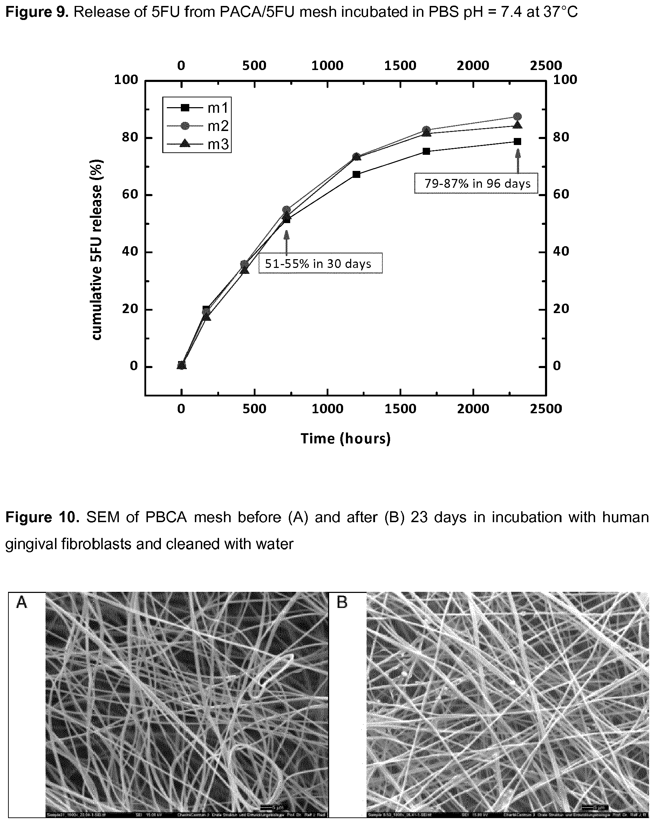

Consistent drug release pattern up to 51-55% was observed in 30 days in PBS media. Figure 10 shows the release of 5FU from PACA/5FU 15% mesh incubated in PBS pH = 7.4 at 37°C. At day 96 the drug release was 79-87% of the total drug content for all samples. See Figure 9. Multilayered electro-spun biodegradable meshes composed of PACAs random nano/microfibers can be provided, charged with any type of animal cells, comprising human cells which could belong to the own subject receiving the treatment. These meshes can effectively serve for tissue engineering.

As described elsewhere herein, the PACA polymer concentration, in particular the PBCA concentration, may be between 0.5 to 50% w/w, preferably in acetone or acetone/ethanol mixtures. In various embodiments, the PACA is a PACA homopolymer, in particular a PBCA homopolymer, with a **low molecular weight**, *e.g*., 10³ Da, and the polymer concentration (PACA, in particular PBCA) is in the range between 20% w/w and up to 50% w/w in the solution. Preferably, the said molecular weight denotes the number-average molecular weight. In various preferred embodiments, the solvent is acetone or an acetone/ethanol mixture. Preferably, the polymer concentration (PACA, in particular PBCA) of the low molecular weight polymer is in the range between 30% w/w and up to 50% w/w, wherein the solvent preferably is acetone or an acetone/ethanol mixture. More preferably, the polymer concentration (PACA, in particular PBCA) of the low molecular weight polymer is in the range between 35% w/w and 50% w/w, wherein the solvent preferably is acetone or an acetone/ethanol mixture. Still more preferably, the polymer concentration (PACA, in particular PBCA) of the low molecular weight polymer is in the range between 40% w/w and 50% w/w, wherein the solvent preferably is acetone or an acetone/ethanol mixture. In particularly preferred embodiments, the low molecular weight polymer concentration (PACA, in particular PBCA) is about 50% w/w, wherein the solvent preferably is acetone or an acetone/ethanol mixture. Such embodiments can provide for more viscous PACA solutions.

As disclosed herein, fibers with different cross-sections can be produced depending on the solvent used. In particular, the methods of the present invention allow for processing PACA materials into different nanofibers morphologies, including ribbon (flat) or full cylinder like (round) without alteration of the PACA chemical structure. For example, round fibers can be produced when acetone/ethanol is used, and flat fibers can be produced when acetone is used alone.

The nano/microfiber meshes are advantageously formed from PACA materials since PACAs are biodegradable, *e.g*., degradable by the action of various enzymes such as esterases in the normal functioning of the human body and living organisms where the byproducts are the corresponding alkyl alcohol and polycyanoacrylic acid, which are water soluble substances and removed *in vivo* by renal filtration. The meshes can thus be used in various applications where the mesh completely degrades within the body over time without the requirement to remove the mesh after use, for example by an additional surgery. Especially for dental applications, omitting a second surgery for removal of the mesh is advantageous to ensure a better healing process of the treated tissue. In addition, PACAs are generally bacteriostatic and may stop the growing of gram-positive and some gram-negative bacteria. PACAs are also hemostatic. The implanted mesh may thus be used to cause mechanical blockage to slow blood flow, or to provide a surface agent to activate the clotting cascade. The porous mesh may advantageously be invaded with blood with subsequent clotting within the pores of the mesh when used as a tissue adhesive.

In some aspects, the instant disclosure further provides **methods for treating or preventing diseases or disorders**, or for maintaining a healthy or good condition, or the like, for example by local administration of the novel ready-to-use poly(alkyl cyanoacrylate) based nano/microfibers as described herein. For example, in some embodiments, the disclosure provides methods of treating an external wound, the method comprising: topically administering to an external wound of a subject the novel ready-to-use poly(alkyl cyanoacrylate) based nano/microfiber as disclosed herein. As another example, the disclosure provides methods of treating an external burn, the method comprising: topically administering to an external burn of a subject the novel ready-to-use poly(alkyl cyanoacrylate) based nano/microfibers as disclosed herein. As another example, the disclosure provides methods of treating aches and pains, the method comprising: topically administering to a skin area of a subject the novel ready-to-use poly(alkyl cyanoacrylate) based nano/microfibers as disclosed herein, wherein the skin area preferably is proximate to (*e.g*., immediately above) the ache or pain. As another example, the disclosure provides methods of promoting gum health (e.g., by inhibiting the growth of oral bacteria), the method comprising: orally administering to a subject the novel ready-to-use poly(alkyl cyanoacrylate) based nano/microfibers as disclosed herein. As another example, the disclosure provides methods of treating peri-implantitis, the method comprising: orally or dentally administering to a subject the novel ready-to-use poly(alkyl cyanoacrylate) based nano/microfibers as disclosed herein.

In some embodiments of the present invention, ready-to-use PACA-based nano/microfibers contemplated herein may be suitable for uses such as cosmetic and/or personal care applications to: (i) cover and protect as a film-forming ingredient to favor moisture control; (ii) provide antimicrobial activity as a natural preservative; and/or (iii) act as a carrier of other active ingredients for slow release, due to ionic bonding to compounds and chitosan degradation by natural skin enzymes.

In some embodiments of the present invention, ready-to-use PACA-based nano/microfibers contemplated herein may be applied in a variety of ways, for example, to: (i) cover, seal and/or protect a wound or injured/inflamed skin or area; (ii) result in a smooth and tailor-made film, easily shaped to the skin or area under treatment, that may be degraded by skin enzyme(s); (iii) provide the appropriate conditions for moisture control; (iv) control skin and wound infections; (v) reduce itching and inflammation; (vi) contribute to rapid healing; and/or (vii) reduce hypertrophic scar formation; (viii) relieve aches and pains related to sore muscles and joints; and/or (ix) treat disorders within the oral mucosa and tooth supporting tissues. Additional uses contemplated herein include the targeted cleaning of dirty wounds, udder care for milking animals, treatment of human and animal wounds or burns, to calm skin inflammation, relieve muscle aches and pains and/or to reduce itching due to insect bites or plant poison, sunburns, bedsores, ulcers, to promote oral health, *e.g*., by inhibiting the growth of oral bacteria, and to treat other disorders of the oral mucosa and the tooth-supporting tissues, and the like. Ready-to-use PACA-based nano/microfibers contemplated herein have a variety of advantages, *e.g*., a mesh of nano/microfibers will create a thicker film that lasts longer than a spray solution; such materials are less prone to rapid biodegradation, and can be more easily adapted to a wound than a bandage, just covering the area needed, tailor-made transparent and flexible "bandage" with various properties.

In some aspects, the disclosure provides methods of promoting oral health (*e.g*., promoting gum health), comprising orally applying (or administering) to a subject any of the ready-to-use PACA-based nano/microfibers disclosed herein to the soft or hard tissues of the mouth. In an analogous embodiment, the disclosure provides any of the ready-to-use PACA-based nano/microfibers disclosed herein for use in promoting oral health (*e.g*., promoting gum health). In another analogous embodiment, the disclosure provides uses of any of the ready-to-use PACA-based nano/microfibers disclosed herein for the manufacture of a medical device or medicament for promoting oral health (*e.g*., promoting gum health). The disclosure provides methods of treating disorders of the oral mucosa and/or the tooth supporting tissues, such as treating peri-implantitis, comprising orally applying or administering to a subject any of the ready-to-use PACA-based nano/microfibers disclosed herein.

The present invention specifically encompasses the use of the novel PACA-based nano/microfibers and/or the PACA-based nano/microfiber meshes disclosed herein for use in dental and/or oral applications, for wound dressing/healing, tissue regeneration/engineering, organ protection, implant's coating, or controlled drug delivery. The use of the novel PACA-based nano/microfibers and/or the PACA-based nano/microfiber meshes of the present invention in dental and/or oral applications are preferred embodiments.

The present invention specifically encompasses the use of the novel composite materials disclosed herein for use in dental and/or oral applications, for wound dressing/healing, tissue regeneration/engineering, organ protection, implant's coating, or controlled drug delivery. The use of the novel composite materials of the present invention in dental and/or oral applications are preferred embodiments. In various preferred embodiments, the composite material comprises (i) PACA-based nano/microfibers or PACA-based nano/microfiber meshes disclosed herein, and (ii) chitosan.

A **patient** or subject to be treated by any of the novel ready-to-use poly(alkyl cyanoacrylate) based nano/microfibers or methods of the present disclosure can mean either a human or a non-human animal. In an embodiment, the present disclosure provides methods for the treatment of a disease in a human patient in need thereof. In an embodiment, the present disclosure provides methods for the treatment of an inflammatory disease in a human patient in need thereof. In another embodiment, the present disclosure provides methods for the treatment of a disease in a veterinary patient in need thereof, including, but not limited to dogs, horses, cats, rabbits, gerbils, hamsters, rodents, birds, aquatic mammals, cattle, pigs, camelids, and other zoological animals.

The term "**treating**" refers to: preventing a disease, disorder or condition from occurring in a cell, a tissue, a system, animal or human which may be predisposed to the disease, disorder and/or condition but has not yet been diagnosed as having it; stabilizing a disease, disorder or condition, *i.e*., arresting its development; and/or relieving one or more symptoms of the disease, disorder or condition, *i.e*., causing regression of the disease, disorder and/or condition. As described herein, a therapeutic that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample. Accordingly, in some aspects and embodiments of the present disclosure, there are provided methods of treating or preventing a disease or condition, that includes locally administering the novel ready-to-use poly(alkyl cyanoacrylate) based nano/microfibers as disclosed herein.

The words "comprises/comprising" and the words "having/including" when used herein with reference to the present invention are used to specify the presence of stated features, integers, steps or components but do not preclude the presence or addition of one or more other features, integers, steps, components or groups thereof. In various embodiments, the term "comprising" may encompass the term "consisting of".

Furthermore, the term "about" is understood by persons of ordinary skill in the art in the context in which it is used herein. In particular, "about" is meant to refer to variations of ±20%, ±10%, preferably ±5%, more preferably ±1 %, and still more preferably ±0.1 %, of the indicated value. Also, it is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

The present disclosure will now be described in detail with reference to the following examples. However, these examples are only for illustrative purposes and are not intended to limit the scope of the present disclosure.

### EXAMPLES

In the following examples, the obtained polymeric solutions and emulsions were placed in a horizontally oriented 10 mL plastic syringe fitted with needles with different internal diameters from 25G to 15G. Different collectors were used: copper meshes, copper rings, aluminum foil, paper and stainless steel squares. Those collectors were grounded and were positioned at a distance of 5 to 30 cm away from the needle. The voltages applied to the needle ranged from 5-30 kV. Flow rates ranged from 0.025 to 2.000 mL/min. Scanning electron microscopy (SEM) was used to confirm the morphology of the obtained meshes. Pore characteristics and average fiber diameters of the nonwoven meshes were measured from the SEM pictures using ImageJ software, version 1.51n (http://imagej.nih.gov/ij). First, meshes based on PACAs electro-spun nano/micro-fibrous were sputter coated with gold for two minutes using a Balzers Sputter Coater SCD 040 (Manchester, NH.) and the sputter coated meshes were observed using Maxim (Cambridge, UK) scanning electron microscope at a working distance between 18-30 mm, and an accelerating voltage of 15-20 kV.

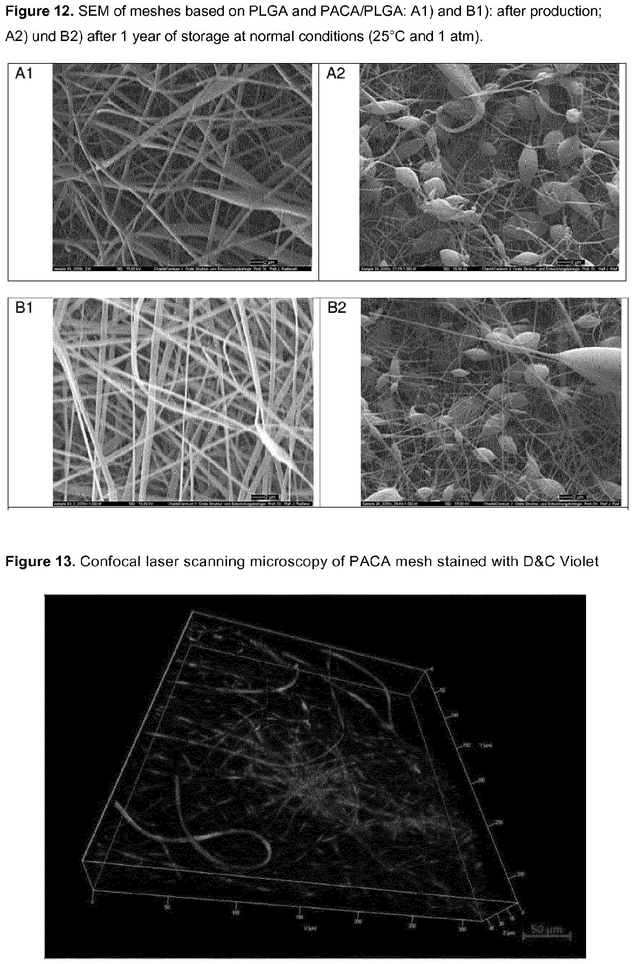

Ribbon like electro-spun fibers (Figure 12, A1) and full cylinder like electro-spun fibers (Figure 6, B1) were obtained generally by preparing solvent mixtures in different relations. Figure 12 is a SEM picture of meshes based on PACA at the time of production ("year 0"), A1) flat fibers and B1) round fibers; and after one year of storage at normal conditions, A2) and B2). Fiber diameters were regulated mainly by the concentration of PACAs based solutions (0.1 to 70% (w/w)), the lower ones for PACAs with number average molecular weight between 10⁵ to 10⁶ Da and the bigger ones for PACAs with the lower number average molecular weights (≈10³-10⁴ Da). Figure 13 is a SEM picture of meshes based on PLGA and PACA/PLGA measured at the time of production ("year 0"), A1) and B1); and after one year of storage at normal conditions, A2) and B2).

### Example 1

### Electrospinning of PHCA - poly(hexyl cyanoacrylate)

A mixture of acetone/ethanol 2/1 (v/v) was prepared. PHCA with number average molecular weight in the order of 10⁶ Da was dissolved in the solvent mixture using magnetic stirring to prepare 0.1% (w/w) solution. The solution is placed inside a plastic 10 mL syringed fitted to a stainless-steel needle tip 25G. A collector (a square copper mesh of 100 cm²) is placed at a distance of 10 cm of the needle tip. The fluid rate is fixed at 0.125 mL/min, and 10 kV are applied for a period of 30 minutes. Cylinder shaped full nanofibers are obtained with an average fiber diameter of 696 ± 113 nm, a porosity of 54% and a thickness below 500 pm. The mechanical properties are summarized in Table 1, with a Young's modulus of 1.2 MPa and a tensile strength of 0.08 MPa.

### Example 2

### Electrospinning of POCA and PLGA (POCA = poly(octyl cyanoacrylate))

A blend of POCA and PLGA powders with a PLGA maximum concentration up to 50% was dissolved in ethyl acetate. A 1/1 ratio of POCA and PLGA with number average molecular weight in the order of 10⁶ Da was prepared by two alternative ways:
1) preparing two polymeric solutions independently, one with a POCA concentration of 0.5% and one with a PLGA concentration of 12%, followed by mixing them;
2) preparing the PLGA solution; after the PLGA was completely dissolved, POCA was added, followed by stirring until the added POCA was completely dissolved.

The obtained solution was placed inside aplastic 10 mL syringe fitted to a stainless steel needle tip 17G. A collector (a square shaped aluminum foil of 10 cm²) was placed at a distance of 7 cm of the needle tip. The fluid rate was fixed at 0.5 mL/min. 20 kV were applied for a duration of 30 minutes. A mixture of cylinder shaped and ribbon like nanofibers was obtained, with a fiber diameter around 900 nm. Theses meshes are thicker than those based on POCA alone, but with a diameter of less than 500 µm. The porosity of the mesh was measured to be about 39%. The Young's modulus of the meshes was about 1 MPa and the tensile strength was about 0.06 MPa.

### Example 3

### Electrospinning of POCA and PIBCA-co-chitosan

Meshes based on PACAs and chitosan were produced in two alternative ways:
1) from an emulsion obtained by dissolving chitosan with a weight average molecular weight in the order of 10⁶ Da at 1 % in acetic acid at 0.1 mol/L, and separately preparing a solution of isobutyl cyanoacrylate (IBCA) at 2% in acetone. The IBCA solution was added to the chitosan solution, maintaining the relation IBCA/chitosan equal to 60/40 (w/w). Acetone was added to the emulsion to dilute it when the polymerization finished. The emulsion was under stirring overnight. One drop of the dense part of the emulsion was diluted in butanol, this fresh emulsion was used for electro-spinning (Voltage = 15 kV; needle tip-collector distance = 5 cm; needle tip = 18G; fluid rate = 0.250 mL/min).
2) POCA was mixed with the copolymer PIBCA-co-chitosan (produced in the afore-mentioned emulsion system), maintaining the relation POCA/PIBCA-co-chitosan 70/30 w/w where the total polymer percent in acetone was 1%.

The mesh produced by alternative 1) resulted in a structure similar to a sponge with some particles with diameters between 0.9 and 8 mm (Figure 6, E). The mesh produced by alternative 2) had a thickness around 400 µm with a fiber diameter of about 1 µm, a porosity of 69%, a Young's modulus of about 1 MPa and tensile strength of about 0.07 MPa.

### Example 4

### Electrospinning of PPCA and a drug (PPCA = poly(n-pentyl-2-cyanoacrylate)

A solution of 5FU in formic acid is prepared. PPCA with a molecular weight of 10⁵ Da was added to obtain a 2% (w/w) solution, with 5FU being 15% with respect to the weight of the PPCA. The polymeric solution was placed inside a plastic 10 mL syringe fitted to a stainless steel needle tip 21G. A collector (square shaped stainless steel of 1 cm² over an aluminum foil) was placed vertically at a distance of 20 cm of the needle tip. The fluid rate was fixed at 0.05 mL/min, and 22 kV were applied. A mixture of cylinder shaped nano/microfibers and ribbon like nano/microfibers was obtained with a mean fiber diameter equal to 798 ± 310 nm and a porosity of 60%. The thickness of the mesh was about 390 pm with a Young's modulus of about 0.7 MPa and a tensile strength of about 0.1 MPa.

### Example 5

### Electrospinning of PBLCA - poly(butyl-lactoyl-2-cyanoacrylate)

PBLCA with a number average molecular weight in the order of 10³ Da was dissolved in a solvent mixture of dimethyl formamide/acetone = 1/2 (v/v) using magnetic stirring to prepare a 50% (w/w) solution. The obtained solution was placed inside a plastic 10 mL syringe fitted to a stainless steel needle tip 15G. A collector (a square shaped aluminum foil of 10 cm²) was placed at a distance of 5 cm of the needle tip. The fluid rate was fixed at 2 mL/min and 13 kV were applied over a time period of 30 minutes. Cylinder shaped and very well connected fibers were obtained (Figure 6A) with an average fiber diameter of 1320 ± 721 nm, 41 % of porosity and a mean pore area of 11 µm².

### Example 6

### Electrospinning of a PHepCA-D&C Violet #2 solution (PHepCA = poly(n-heptyl-2-cyanoacrylate)

PHepCA and D&C Violet #2 solutions were prepared independently in acetone/ethanol = 2/1 and acetone, respectively. The D&C Violet #2 solution was at 8 pg/mL and the PHepCA solution (with a weight average molecular weight in the range of 10⁴ Da) was at 20% (w/w). One drop of the stain solution was added to the PHepCA solution and stirred until the final solution was homogeneous. The solution was placed inside a plastic 10 mL syringe fitted to a stainless steel needle tip 20G. A collector (copper rings with diameters of 1 cm) was placed at a distance of 15 cm of the needle tip. The fluid rate was fixed at 1 mL/min and 11 kV were applied over a period of 30 minutes. Nanofibers were obtained in different layers. The obtained multilayer assembly was confirmed with an LSM 700 Laser Scanning Microscope (Carl Zeiss Microscopy GmbH, 07745 Jena, Germany) in fluorescence mode. Figure 13 illustrates confocal laser scanning microscopy to PACA mesh stained with D&C Violet #2.

### Example 7

### Electro-spun PBCA scaffolds for Cell Culture (support for culturing/growing cells, in particular human cells)

Meshes were obtained from electrospinning a solution of PBCA with a weight average molecular weight of 10⁶ Da 1% in acetone/ethanol = 3/2 (v/v), at feed rate 0.025 mL/min, 5 kV, a collector distance of 5 cm using a copper mesh with stainless steel squares stuck on as the collector. These meshes were composed of PBCA nano/microfibers with a solid cylinder shape with an average diameter of 1.0 ± 0.4 µm.

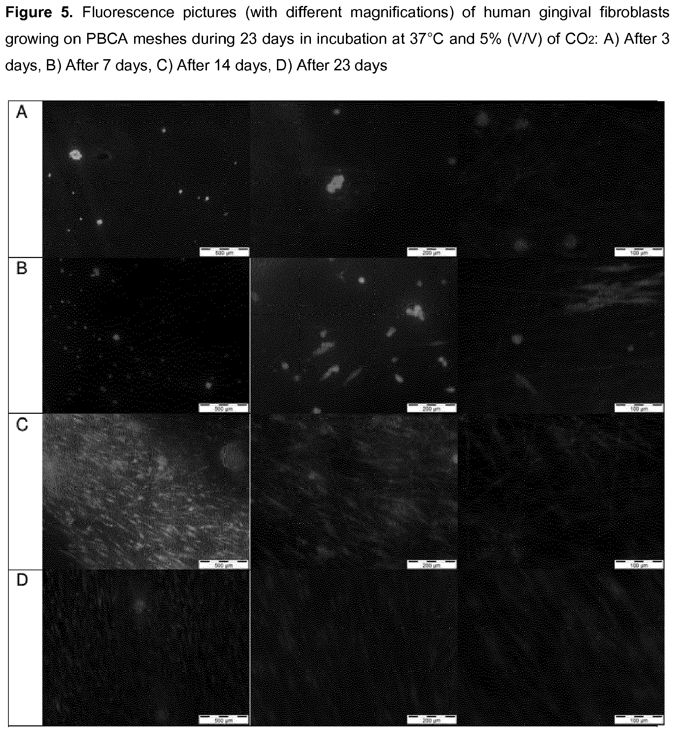

The meshes were used for different experiments. Figure 5 shows fluorescence pictures of human gingival fibroblasts growing on PBCA meshes during 23 days in incubation at 37°C and 5% (V/V) of CO₂. The analyses were made after 3, 7, 14 and 23 days of HGFib incubated over PBCA meshes. As it can be seen, after 7 days cells spread, and after 14 days until 23 days more than 70% of the mesh area was occupied by live and spread cells.

Cell attachment as a result of seeding was determined as follows: in order to assay the cells attached, the medium was removed after the incubation days (3, 7, 14 and 23) and live/dead staining was performed using fluorescein and ethidium bromide respectively to obtain fluorescence pictures using Olympus fluorescent microscope, VANOX-T. Fluorescence pictures (see Figure 5) show that meshes composed of PBCA are able to cultivate human gingival fibroblasts.

### Example 8

### Measurement of mechanical properties of meshes

Mechanical properties of samples were measured using uniaxial tensile testing (Zwick/Z010, Germany, equipped with a 1000 N load cell). Specimens were prepared by cutting the samples into rectangular strips of 10 mm width x 13 mm length (a gauge length of 10 mm) in a thickness range of from 300-400 µm. The crosshead speed was set at 10 mm/min and the analyses were performed at ambient conditions. At least three samples were analyzed for each type of nanofibrous meshes. Tensile strength and Young's modulus was derived from the stress-strain curves generated from the data of force vs. elongation measured by the testing machine (Table 2).

### Example 9

### Measurement of contact angle of meshes

Surface wettability of electro-spun meshes was detected by distillated water contact angle method at room temperature. The water contact angles were measured by dropping water over meshes (at least over 3 different places) and taking immediately one photo with an optic microscopy (KEYENCE VHX-5000, Germany) followed by image processing of sessile drop with KEYENCE software (Table 1).

### Example 10

### Use of the mesh as a membrane

A nanofiber mesh of PBCA was obtained as outlined above. After closing a bone defect, the membrane was formed of the PBCA mesh by cutting it into the required shape and placed as a membrane in between the hard and soft tissue.

### Example 11

A nanofiber mesh of PBCA was obtained as outlined above. The mesh was formed into the required shape and used as a sleeve for a dental implant.

### Example 12

The surface of a biodegradable Mg alloy biomaterial was coated with PBCA nanofiber mesh obtained as outlined above. The degradation speed of the Mg alloy could thus be controlled, thereby reducing the hydrogen production and thus influencing the degradation. Also, a nearly steady degradation speed could be achieved.

### Example 13

Testing for cell attachment and proliferation (potential tissue regeneration/engineering scaffold utility) was carried out as follows:
First, human gingival fibroblasts were plated in a monolayer in two 25 cm² tissue culture flasks and cultured till the cells reached five passages. After the fifth passage, the cells were removed by trypsin treatment, counted, and seeded onto the meshes at a density of 37,500 cells/cm². Meshes were first exposed for 30 minutes to ultraviolet germicidal irradiation to inactivate possible microbiological contaminants that may have arisen during the electrospinning process (sterilization procedure, Figure 9). Cells in contact with meshes were maintained in an incubator at 37° C with 5% CO₂. The medium was changed every three days.

### Example 14

Production of PACA by anionic mass polymerization - Synthesis of PBCA with molecular weight greater than 10³ Da:
PBCA with M̅ₙ̅ in the order of 10⁴, 10⁵ and 10⁶ Da were produced by bulk anionic polymerization of BCA with DMPT as initiator. The reaction was carried out in a three-neck round-bottom flask over cold water bath. BCA was added into the flask first followed by the initiator (diluted in acetone). The flask was gentle stirred. At the end of the polymerization (solid formation, cold reaction system and without visual changes) the solid was solved, precipitated on distilled water, filtrated, washed with water first and methanol later and dried in vacuum. Table 1 shows the identification of the PBCA with the value of their M̅ₙ̅ and the molar relation between BCA and DMPT.

**Table 1. Experimental conditions and results of BCA bulk anionic polymerization.**

| PBCA | BCA:DMPT | **M̅ₙ̅** [Da] | **M̅_̅{̅w̅}̅**/**M̅ₙ̅** |
|---|---|---|---|
| AM | 4·10³ :1 | 1.1·10⁶* ; 8.0-103 | 2.7; 2.7 |
| IAM | 2·10³ :1 | 2.5·10⁵ | 4.5 |
| IIAM | 2·10² :1 | 7.4·10⁴ | 1.7 |
| IIIAM | 1·10³ :1 | 1.2·10⁴ | 1.4 |

| | | | |
|---|---|---|---|
| *polymeric population in higher proportion. AM, IAM, IIAM, IIIAM: designation of different polymers. | | | |

### Example 15

### Meshes based on PBCA alone:

PBCA with different molecular weights could be electro-spun, from high molecular weight (10⁶ Da) to low molecular weight (10³ Da), with polymer concentrations from 0.5 to 50% w/w in acetone or acetone/ethanol mixtures, respecting the molecular weight of the PBCA. The thickness of the meshes was around 260 µm. The fiber diameter of the meshes increases with the concentration of the polymer. For example, meshes based on PBCA nanofibers (600 nm) were obtained with 0.5% of polymer solution (PBCA high molecular weight), while microfiber
meshes (around 1 µm) were obtained with 1.1% w/w of polymer solution (PBCA high molecular weight). Surprisingly it was tested that continuous and smooth fibers could be obtained using these very low viscous PBCA solutions (0.3-3 cP), something that it is not possible using other polymers like PCL. In the case of PBCA with low molecular weight, viscous solutions (up to 50% w/w) were used for electro-spinning. Fibers with different cross sections can be produced, round when acetone/ethanol is used, and flat fibers when acetone is used alone. The pore area of the meshes is in the range of 0.1-370 µm². The mechanical properties of the meshes composed by PBCA of high molecular weight do not differentiate too much if the fibers are flat or round, neither with the age of the mesh (at least 1 year), nor with the range of fiber diameters tested (600 nm and 1.5 µm). The Young's modulus of those meshes was around 1.2 MPa, the tensile strength was around 0.08 MPa, and the failure strain was around 15%; values that are closer to elastic materials than rigid materials.

### Example 16

### Meshes based on PBCA-PLGA:

This mesh was produced by electrospinning a blend solution of PBCA high molecular weight and PLGA (75:25 high molecular weight powders with PLGA maximum concentration up to 50% in acetone with a maximum concentration of PBCA 1% w/w). Two ways were used to prepare the blend solution: 1) preparing two polymeric solutions in acetone independently, one of PBCA 1% and the other of PLGA 75:25 12%, and then mixing them; and 2) preparing the PLGA acetone solution and after the PLGA is solved, adding the PBCA and stirring until PBCA is completely solved. These meshes were thicker (around 390 µm) than those based in PBCA alone. The fiber diameter was around 900 nm. The pore area of the meshes was in the range of 0.1-160 µm². The Young's modulus of the meshes was around 1 MPa, the tensile strength was around 0.06 MPa and the failure strain was around 20%.

### Example 17

### Meshes based on PBCA-Chitosan

Meshes based on PBCA and chitosan were produced by mixing PBCA of high molecular weight with a copolymer of PBCA-chitosan (powder) produced in the emulsion system described before, keeping the relation PBCA/PBCA-chitosan 50/50 w/w, where the total polymer percent in acetone was 2%. The mesh produced had a thickness around 400 µm with fiber diameters around 1 µm, minimum pore area of 0.1 µm², maximum pore area of 482 µm², Young's modulus around 1 MPa, tensile strength around 0.07 MPa and 14% of failure strain.

### Example 18

### Meshes based on PBCA-5FU

Different concentrations of the drug 5FU were incorporated in the polymer solutions to electrospinning: 7, 10, 15, 20, 25 and 30% in relation with the mass of PBCA of high molecular weight, which was used in 1 and 2% acetone. In the way the drug concentration increases, the fiber diameter increases too (700 nm to 1.2 µm), indicative that the drug is incorporated homogeneously inside the fibers, which was tested in the kinetic release assay, where a consistent drug release pattern was observed until 98 days of incubation at 37°C in PBS media. The mesh composed of PBCA of high molecular weight and 5FU at 15% has a thickness around 390 µm, was tensile tested, giving Young's modulus around 0.7 MPa, tensile strength around 0.1 MPa, and 25% of failure strain.

### Example 19

### Meshes based on PBCA-PLA-Mg

The mesh composed of PBCA of high molecular weight with nanoparticles of magnesium encapsulated in particles of poly (lactic acid) was produced by electro-spinning of one emulsion formed by the addition of PLA-Mg powder to one ready PBCA acetone solution, keeping the relation PBCA/PLA-Mg equal to 50/50 or 75/25 (w/w), and the percent of polymer at 2 and 3% (w/w), respectively, in acetone. The fiber diameter of the mesh produced using 2% of the polymer concentration was around 360 nm, and when 3% was used the diameter increases to 1 µm.

## Claims

1. A method of producing ready-to-use poly(alkyl cyanoacrylate) based nano/microfibers, the method comprising:
(a) providing a poly(alkyl cyanoacrylate) homopolymer and/or poly(alkyl cyanoacrylate) copolymer obtained by anionic polymerization of one or more alkyl cyanoacrylate (ACA) monomers and/or one or more ACA oligomers, wherein the alkyl substituent of the ACA monomers and/or the ACA oligomers may optionally be substituted, and wherein the ratio of the weight-average molecular weight (M_{w}) to the number-average molecular weight (Mₙ) of the poly(alkyl cyanoacrylate) homopolymer and/or poly(alkyl cyanoacrylate) copolymer is from 1 to 1.7;
(b) dissolving the poly(alkyl cyanoacrylate) homopolymer and/or poly(alkyl cyanoacrylate) copolymer of step (a) in a solvent;
(c) electrospinning the solution obtained in step (b) to obtain poly(alkyl cyanoacrylate)-based nano/microfibers on a collector; and
(d) removing the poly(alkyl cyanoacrylate) based nano/microfibers from the collector to obtain ready-to-use poly(alkyl cyanoacrylate) based nano/microfibers.

2. The method of claim 1, wherein the poly(alkyl cyanoacrylate) copolymer in step (a) comprises (i) at least two different ACA monomer species, (ii) at least two alkyl cyanoacrylate oligomers comprising at least two different ACA monomer species, or (iii) at least one poly(alkyl cyanoacrylate) (PACA) polymer and at least one non-PACA polymer.

3. The method of claim 1 or 2, wherein the one or more ACA monomers, or the at least two different ACA monomer species, are selected from any of n-butyl-2-cyanoacrylate (BCA), iso-butyl-2-cyanoacrylate (IBCA), n-pentyl-2-cyanoacrylate (PCA), iso-pentyl-2-cyanoacrylate (IPCA), n-hexyl-2-cyanoacrylate (HCA), iso-hexyl-2-cyanoacrylate (IHCA), cyclo-hexyl-2-cyanoacrylate (CHCA), n-heptyl-2-cyanoacrylate (HepCA), iso-heptyl-2-cyanoacrylate (IHepCA), n-octyl-2-cyanoacrylate (OCA), iso-octyl-2-cyanoacrylate (IOCA), butyl-lactoyl-2-cyanoacrylate (BLCA), and mixtures thereof.

4. The method of claim 1 or 2, wherein the one or more ACA oligomers are selected from oligomers of any of n-butyl-2-cyanoacrylate (BCA), oligomers of iso-butyl-2-cyanoacrylate (IBCA), oligomers of n-pentyl-2-cyanoacrylate (PCA), oligomers of isopentyl-2-cyanoacrylate (IPCA), oligomers of n-hexyl-2-cyanoacrylate (HCA), oligomers of iso-hexyl-2-cyanoacrylate (IHCA), oligomers of cyclo-hexyl-2-cyanoacrylate (CHCA), oligomers of n-heptyl-2-cyanoacrylate (HepCA), oligomers of iso-heptyl-2-cyanoacrylate (IHepCA), oligomers of n-octyl-2-cyanoacrylate (OCA), oligomers of iso-octyl-2-cyanoacrylate (IOCA), oligomers of butyl-lactoyl-2-cyanoacrylate (BLCA), and mixtures thereof.

5. The method of any of claims 1 to 4, wherein the solvent in step (b) is selected from any of acetone, ethanol, acetic acid, formic acid, ethyl acetate, dimethyl sulfoxide, dimethyl formamide, butyl acetate, isobutyl acetate, and mixtures thereof.

6. The method of any of claims 1 to 5, wherein at least one non-PACA polymer is added to the solution obtained in step (b) prior to electrospinning.

7. The method of any one of claims 2 to 6, wherein the at least one non-PACA polymer is selected from any of poly(lactic-co-glycolic acid) (PLGA), poly(ε-caprolactone) (PCL), poly(ethyelenglycol) (PEG), poly(lactic acid) (PLA), chitosan (CH), hyaluronic acid (HA), and dextran (Dex).

8. The method of any of claims 1 to 7, wherein the ready-to-use nano/microfibers obtained in step (d) are not comprised by a support.

9. The method of any of claims 1 to 8, wherein the ratio of the weight average molecular weight to the number average molecular weight is from 1.1 to 1.5; or wherein the ratio of the weight average molecular weight to the number average molecular weight is from 1.2 to 1.4.

10. Ready-to-use nano/microfibers obtainable by the method of any of claims 1 to 9.

11. The ready-to-use nano/microfibers of claim 10, wherein the fiber average diameter is from 100 nm to 5 micrometer.

12. The ready-to-use nano/microfibers of claim 10, wherein the nano/microfibers is immobilized with, or has encapsulated, at least one of a therapeutic agent, antibiotic, antiviral agent, chemotherapeutic agent, analgesic and analgesic combinations, anti-inflammatory agent, vitamin, sedative, radiopharmaceutical, metal particles, metal alloy particles, metal oxide particles, hydroxyapatite, sodium alginate, stains, cells, protein, peptides, nucleic acids, nucleic acid analogs, nucleotides or oligonucleotides, peptide nucleic acids, aptamers, antibodies or fragments or portions thereof, antigens or epitopes, hormones, hormone antagonists, growth factors or recombinant growth factors and fragments and variants thereof, cell attachment mediators, cytokines, enzymes, or a mixture thereof.

13. The ready-to-use nano/microfibers of claim 10, wherein the nano/microfibers further comprise a cell growth medium.

14. Use of the ready-to-use nano/microfibers of claim 10 in dental applications, for wound dressing/healing, tissue regeneration/engineering, organ protection, implant's coating, or controlled drug delivery.

15. A nanofiber mesh comprising the ready-to-use nano/microfibers of any of claims 10 to 13, wherein the surface of the nanofiber mesh is modified chemically and/or physically for biomedical applications, preferably wherein the surface is modified by encapsulation and/or immobilization of a therapeutic agent and/or biological material.
